# EUROPEAN PATENT APPLICATION

(11) **EP 3 471 050 A1**
(43) Date of publication of application: **17.04.2019**
(21) Application number: 17810174.7
(22) Date of filing: 31.05.2017
(51) Int. Cl.: G06Q 50/22, A61B 5/00, G08B 25/04, G08B 25/10

(54) **DEVICE, METHOD, AND SYSTEM FOR MONITORING MONITORED PERSON**

(30) Priority: 09.06.2016 JP 2016115042
(71) Applicant: Konica Minolta, Inc., Tokyo 100-7015 (JP)
(72) Inventor: NISHIKADO, Masashi, Tokyo 100-7015 (JP); ASANO, Masaki, Tokyo 100-7015 (JP); FUJIWARA, Koji, Tokyo 100-7015 (JP)
(74) Representative: Henkel, Breuer & Partner
(86) International application number: PCT/JP2017/020218
(87) International publication number: WO 2017/212995

(57) **Abstract**

With this device, method, and system for monitoring a monitored person, breathing of the monitored person is measured, whether or not the monitored person is breathing normally is determined on the basis of the measurement result, a notification condition is obtained on the basis of the determination result, a notification destination that corresponds to the notification condition is identified as a first notification destination, the first notification destination is selected from among multiple notification destinations that have been classified in accordance with predetermined criteria, and the first notification destination is notified of the determination result. In addition, in the case of the present invention, when an instruction to forward the determination result is received from the first notification destination, another notification destination is selected as a second notification destination from among the multiple notification destinations, and the determination result is resent to the second notification destination.

## Description

### Technical Field

The present invention relates to a monitored person monitoring device, a monitored person monitoring method, and a monitored person monitoring system for monitoring a monitored person who is an object to be monitored.

### Background Art

Japan is in the aging society, more specifically, in the super aged society in which the ratio of the population aged 65 or older to the total population exceeds 21% due to the improvement of standard of living with high economic growth after the war, the improvement of hygienic environment, the improvement of medical standards, and the like. Further, in 2005, the population of the elderly aged 65 or older was about 25.56 million to the total population of 127.65 million whereas in 2020, the population of the elderly aged 65 or older is estimated to be about 34.56 million to the total population of 124.11 million. In such an aging society, the number of care recipients who need nursing and care due to sickness, injury, elderly, and the like is expected to increase more than that in a non-aging society. Further, Japan is also in the society with fewer children where the total fertility rate in 2013 is 1.43, for example. For that reason, an elderly caring for an elderly has occurred in which an elder family member (a spouse, a child, a brother, or a sister) cares for an elder care recipient.

The care recipients and others are put in facilities such as hospitals and elderly welfare facilities (in the laws of Japan, short-term facilities for senior citizens, nursing homes and special nursing homes, and the like) and receive nursing and care. In such a facility, a care recipient or the like may suffer injuries due to rolling off the bed, or falling during walking, or situations such as getting out of the bed and walking around may occur. These kinds of situations need to be dealt with as soon as possible. Such a situation may develop into a more serious situation if neglected. For this reason, at the facility, nurses, caretakers, and the like regularly inspect the safety and confirm the condition.

However, the number of nurses and the like fails to catch up with the increased number of care recipients, and the nursing industry and the nursing care industry chronically face labor shortage. Furthermore, as the number of nurses and caretakers in the twilight and evening hours decreases as compared with those in the daytime hours, work load per person increases, so reduction of the workload is demanded. In addition, the situation of elderly caring for elderly is not an exception at the facility, and elder nurses and the like often care for elder care recipients. Generally, the physical strength will wane as people get older, so even if they are healthy, the burden of nursing care is heavier than young nurses, and their movements and judgment become slow.

To alleviate such labor shortage and burden of nurses and others, technologies complementing nursing services and nursing care services are required. For this reason, in recent years, monitored person monitoring technologies for monitoring monitored persons who are persons to be monitored such as the care recipients have been researched and developed.

As one of such techniques, for example, a cardiopulmonary health state monitoring device disclosed in Patent Literature 1 includes a non-contact motion sensor configured to generate one or more motion signals indicating physical movement of a patient during a monitoring session, a processor, and a memory for storing program commands configured to cause the processor to perform a procedure for processing the one or more motion signals. The procedure includes extracting one or more sleep disordered respiration characteristics from the one or more motion signals, and predicting whether a clinical event occurs within a predetermined prediction range on the basis of the one or more sleep disordered respiration characteristics.

By the way, since monitored persons such as care recipients are placed in the facilities as described above, they will be greeted at the facilities at the last moment (deathbed). For this reason, monitors such as the nurses and caretakers need to make a contact with close relatives of the monitored person, such as family members, at the last moment of the monitored person. However, determination of the last moment of the monitored person is often entrusted to experience, and it is not always possible to carry out the contact properly.

The cardiopulmonary health state monitoring device disclosed in Patent Literature 1 can monitor the cardiopulmonary health condition such as Cheyne-Stokes respiration or heart failure, but is silent about the above-mentioned contact and the like at the last moment.

### Citation List

### Patent Literature

Patent Literature 1: JP 2015-522314 W

### Summary of Invention

The present invention has been made in view of the foregoing, and an object thereof is to provide a monitored person monitoring device, a monitored person monitoring method, and a monitored person monitoring system that can support contact at the last moment of a monitored person.

A monitored person monitoring device, a monitored person monitoring method, and a monitored person monitoring system according to the present invention measure respiration of a monitored person, determine whether the respiration of the monitored person is abnormal on the basis of a measurement result, and obtain a notification condition on the basis of a determination result, select a notification destination corresponding to the notification condition as a first notification destination from among a plurality of notification destinations classified according to predetermined criteria, and notify the first notification destination of the determination result. Then, in the monitored person monitoring device, the monitored person monitoring method, and the monitored person monitoring system according to the present invention, in a case where an instruction to forward the determination result is received from the first notification destination, another notification destination is selected as a second notification destination from among the plurality of notification destinations, and the determination result is re-notified to the second notification destination.

These and other objectives, features, and advantages of the present invention will become clear from the following detailed description and accompanying drawings.

### Brief Description of Drawings

Fig. 1 is a diagram illustrating a configuration of a monitored person monitoring system according to an embodiment.
Fig. 2 is a diagram illustrating a configuration of a sensor device in the monitored person monitoring system illustrated in Fig. 1.
Fig. 3 is a diagram illustrating representative waveform patterns in normal respiration and abnormal respiration.
Fig. 4 is a diagram illustrating a configuration of a management server device in the monitored person monitoring system illustrated in Fig. 1.
Fig. 5 is a diagram illustrating a configuration of a server-side monitoring information table stored in the management server device illustrated in Fig. 4.
Fig. 6 is a diagram illustrating configurations of an inter-device information table stored in the management server device illustrated in Fig. 4.
Fig. 7 is a diagram illustrating a configuration of a server-side sensor information table stored in the management server device illustrated in Fig. 4.
Fig. 8 is a diagram illustrating a configuration of a mobile terminal device in the monitored person monitoring system illustrated in Fig. 1.
Fig. 9 is a flowchart illustrating an operation of the sensor device illustrated in Fig. 2.
Fig. 10 is a flowchart illustrating an operation of the management server device illustrated in Fig. 4.
Fig. 11 is a flowchart illustrating monitoring information processing in the flowchart illustrated in Fig. 10.
Fig. 12 is a flowchart illustrating near-death determination request processing in the flowchart illustrated in Fig. 10.
Fig. 13 is a flowchart illustrating an operation of the mobile terminal device illustrated in Fig. 8.
Fig. 14 is a diagram illustrating an example of a standby screen displayed on the mobile terminal device illustrated in Fig. 8.
Fig. 15 is a diagram illustrating an example of a monitoring information screen in first mode displayed on the mobile terminal device illustrated in Fig. 8.
Fig. 16 is a diagram illustrating an example of the monitoring information screen in second mode displayed on the mobile terminal device illustrated in Fig. 8.
Fig. 17 is a diagram illustrating an example of a nurse call reception screen displayed on the mobile terminal device illustrated in Fig. 8.
Fig. 18 is a diagram illustrating a near-death determination request screen displayed on the mobile terminal device illustrated in Fig. 8.
Fig. 19 is a sequence diagram illustrating an operation example of the monitored person monitoring system illustrated in Fig. 1.

### Description of Embodiments

Hereinafter, an embodiment of the present invention will be described with reference to the drawings. Note that configurations denoted by the same reference numeral in the drawings indicate the same configuration, and description thereof is omitted as appropriate. In the present specification, in the case of collectively referring to a configuration, a reference numeral without a suffix is denoted, and in the case of individually referring to a configuration, a reference numeral with a suffix is denoted.

A monitored person monitoring system according to an embodiment monitors a monitored person (watched person) Ob that is an object to be monitored (object to be watched) that should be monitored (watched), and includes a terminal device and a monitored person monitoring device communicatively connected to the terminal device. The monitored person monitoring device monitors the monitored person Ob and sends a predetermined notification to the terminal device according to a monitoring result. In the present embodiment, a monitored person monitoring device includes a respiration measurement unit provided in association with a monitored person that is an object to be monitored and configured to measure respiration of the monitored person, a respiratory abnormality determination unit configured to determine whether the respiration of the monitored person is abnormal on the basis of a measurement result of the respiration measurement unit, a condition-based notification destination information storage unit configured to store a plurality of notification destinations classified according to predetermined criteria as condition-based notification destination information in association with a predetermined notification condition, and a notification processing unit configured to obtain a notification condition on the basis of a determination result of the respiratory abnormality determination unit, select a notification destination corresponding to the obtained notification condition as a first notification destination from the condition-based notification destination information stored in the condition-based notification destination information storage unit, and notify the selected first notification destination of the determination result of the respiratory abnormality determination unit. In a case where the notification processing unit receives an instruction to forward the determination result of the respiratory abnormality determination unit from the first notification destination to which the determination result of the respiratory abnormality determination unit has been notified, the notification processing unit selects another notification destination excluding the notification destination to which the determination result of the respiratory abnormality determination unit has already been notified as a second notification destination from among the plurality of notification destinations, and performs re-notification processing of notifying the selected second notification destination of the determination result of the respiratory abnormality determination unit. In the above-described monitored person monitoring device, the notification processing unit may perform the re-notification processing to the second notification destination as the first notification destination. Such a monitored person monitoring device may be integrally configured by one device. However, in the present embodiment, the monitored person monitoring device is separately configured by two types of devices by including a sensor device and a management server device (central processing device) communicatively connected with the server device and the terminal device. With the configuration, in a case where there is a plurality of sensor devices, the plurality of sensor devices can be centrally managed by the management server device. This sensor device monitors the monitored person Ob and sends a predetermined notification to the monitoring server device according to a monitoring result, and the management server device resends (re-notifies) the predetermined notification to a predetermined terminal device associated with the sensor device that has sent the predetermined notification. In the present embodiment, the sensor device includes the respiration measurement unit, the respiratory abnormality determination unit, and a respiratory abnormality notification processing unit that notifies the management server device of the determination result of the respiratory abnormality determination unit, and the management server device includes the condition-based notification destination information storage unit and the notification processing unit. Note that the terminal device may be one type of device. However, in the present embodiment, the terminal device includes two types of devices: a fixed terminal device and a mobile terminal device. The main difference between the fixed terminal device and the mobile terminal device is that the fixed terminal device is fixedly operated whereas the mobile terminal device is carried and operated by a monitor (user) such as a nurse or a caretaker. Since these fixed terminal device and mobile terminal device are substantially similar, the mobile terminal device will be mainly described in the following embodiment.

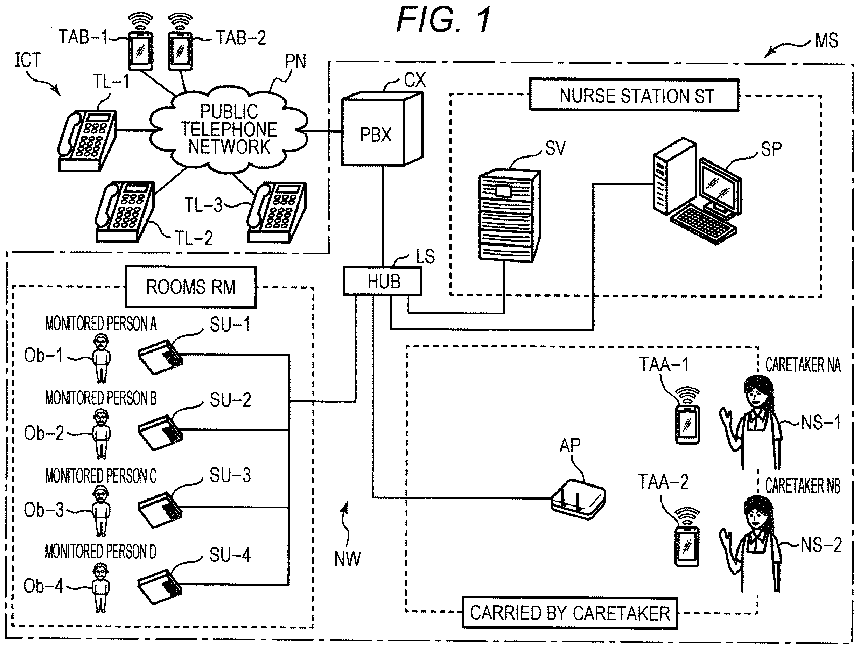

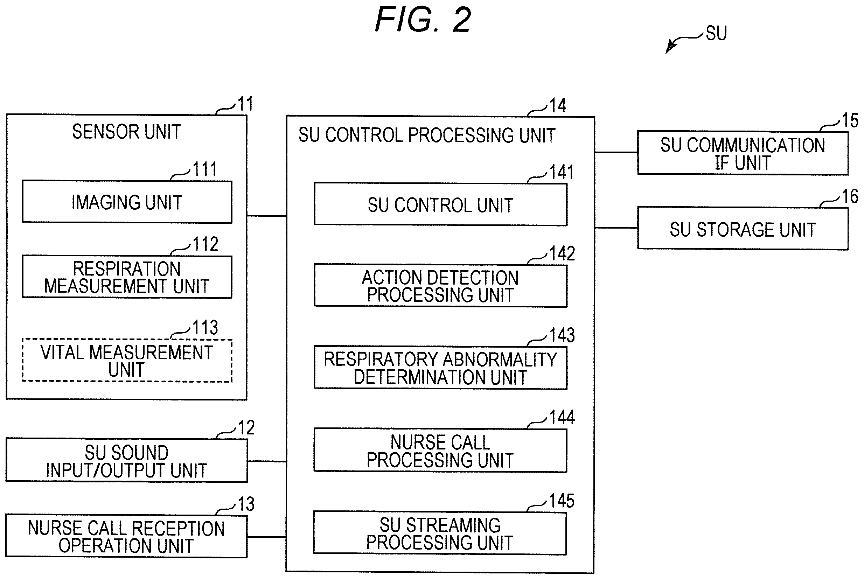

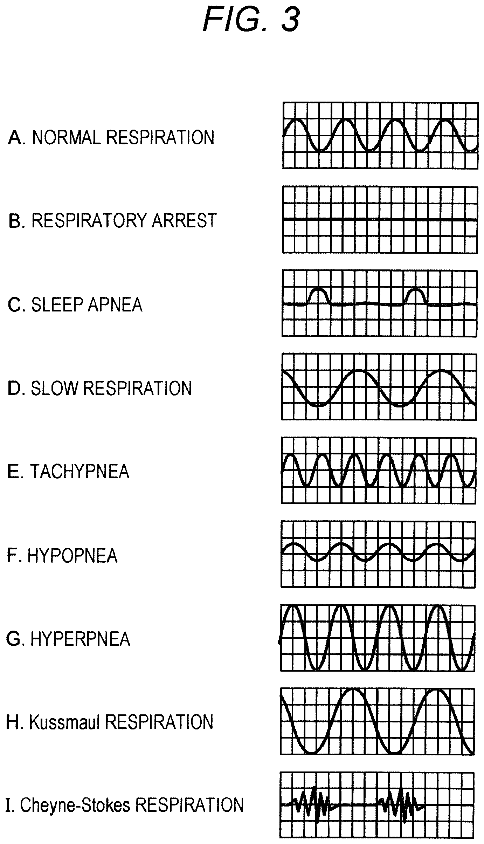

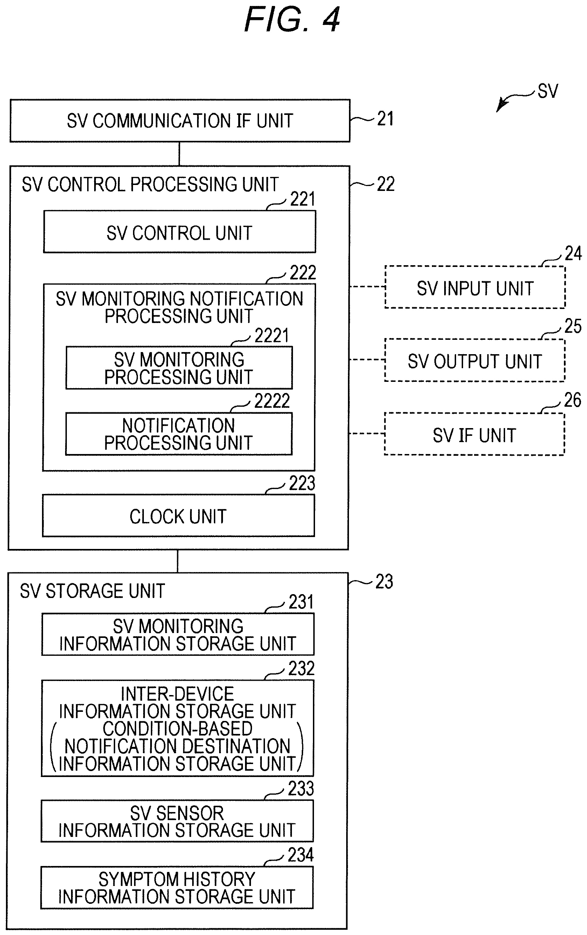

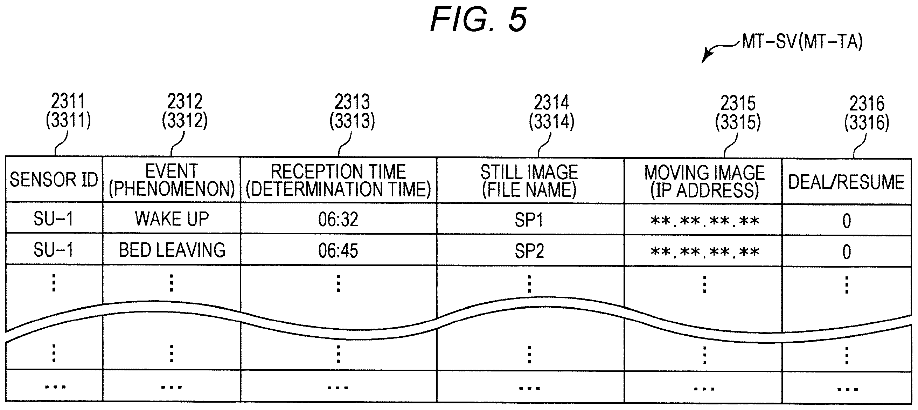

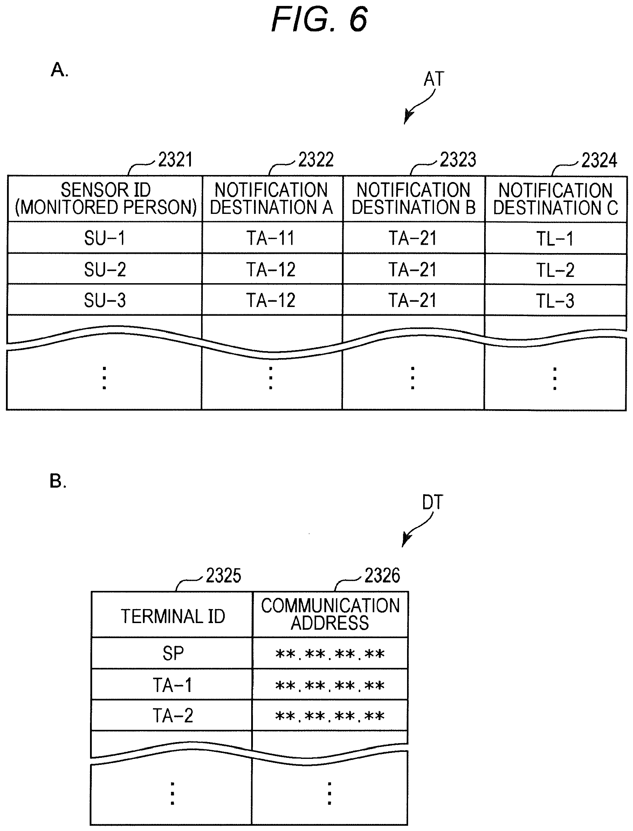

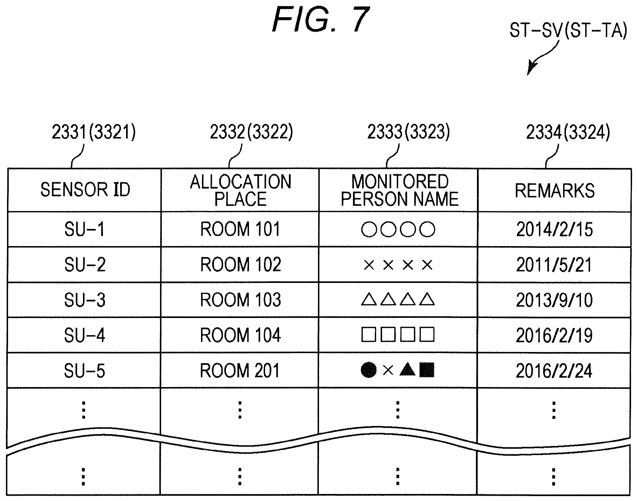

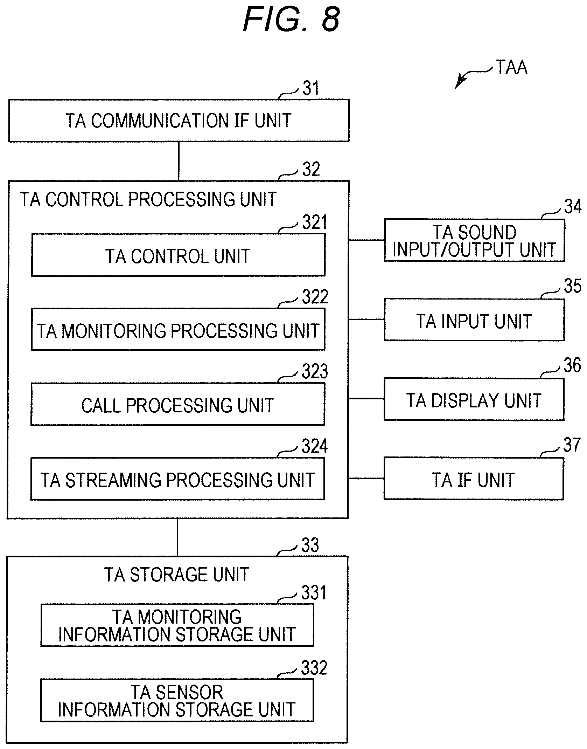

Fig. 1 is a diagram illustrating a configuration of a monitored person monitoring system according to an embodiment. Fig. 2 is a diagram illustrating a configuration of a sensor device in the monitored person monitoring system according to an embodiment. Fig. 3 is a diagram illustrating representative waveform patterns in normal respiration and abnormal respiration. Fig. 3A illustrates a representative waveform pattern in normal respiration, Fig. 3B illustrates a representative waveform pattern in respiratory arrest, Fig. 3C illustrates a representative waveform pattern in sleep apnea, Fig. 3D illustrates a representative waveform pattern in slow respiration, Fig. 3E illustrates a representative waveform pattern in tachypnea, Fig. 3F illustrates a representative waveform pattern in hypopnea, Fig. 3G illustrates a representative waveform pattern in hyperpnea, Fig. 3H illustrates a representative waveform pattern in Kussmaul respiration, and Fig. 3I illustrates a representative waveform pattern in Cheyne-Stokes respiration. Fig. 4 is a diagram illustrating a configuration of a management server device in the monitored person monitoring system according to an embodiment. Fig. 5 is a diagram illustrating a configuration of a server-side monitoring information table stored in the management server device illustrated in Fig. 4. Fig. 6 is a diagram illustrating configurations of an inter-device information table stored in the management server device illustrated in Fig. 4. Fig. 6A illustrates a configuration of a condition-based notification destination correspondence information table in the inter-device information table, and Fig. 6B illustrates a configuration of a communication address correspondence information table in the inter-device information table. Fig. 7 is a diagram illustrating a configuration of a server-side sensor information table stored in the management server device illustrated in Fig. 4. Fig. 8 is a diagram illustrating a configuration of a mobile terminal device in the monitored person monitoring system according to an embodiment.

More specifically, a monitored person monitoring system MS includes, for example, as illustrated in Fig. 1, one or a plurality of sensor devices SU (SU-1 to SU-4), a management server device SV, a fixed terminal device SP, one or a plurality of mobile terminal devices TAA (TAA-1 and TAA-2), and a private branch exchange (PBX) CX, and these devices are communicatively connected to one another via a network (communication line) NW such as a local area network (LAN) in a wired or wireless manner. The network NW may be provided with a repeater such as a repeater, a bridge, or a router, which relays communication signals. In the example illustrated in Fig. 1, the plurality of sensor devices SU-1 to SU-4, the management server device SV, the fixed terminal device SP, the plurality of mobile terminal devices TAA-1 and TAA-2, and the private branch exchange CX are communicatively connected with one another by a wired and wireless mixed LAN (for example, a LAN conforming to the IEEE802.11 standard) NW including an L2 switch concentrator (Hub, HUB) LS and an access point AP. More specifically, the plurality of sensor devices SU-1 to SU-4, the management server device SV, the fixed terminal device SP, and the private branch exchange CX are connected to the concentrator LS, and the plurality of mobile terminal devices TAA-1 and TAA-2 is connected to the concentrator LS via the access point AP. Then, the network NW constitutes a so-called intranet by using a group of Internet protocols such as a transmission control protocol (TCP) and an Internet protocol (IP).

The monitored person monitoring system MS is arranged at an appropriate place according to the monitored person Ob. The monitored person (watched person) Ob is, for example, a person who needs nursing due to sickness or injury, a person who needs nursing care due to a decline in physical ability, or a person who lives alone. In particular, from the viewpoint of enabling early detection and early treatment, the monitored person Ob is favorably a person who needs detection of a predetermined unfavorable phenomenon such as an abnormal state when the event has occurred. Therefore, the monitored person monitoring system MS is suitably arranged in buildings such as a hospital, an aged welfare facility, and a dwelling unit according to the type of the monitored person Ob. In the example illustrated in Fig. 1, the monitored person monitoring system MS is arranged in a building of a nursing facility including a plurality of rooms RM in which a plurality of the monitored persons Ob occupies and a plurality of rooms such as a nurse station.

The private branch exchange (line switching machine) CX is connected to the network NW, and controls extension calls such as outgoing calls, incoming calls, and talks between the mobile terminal devices TAA to conduct the extension calls between the mobile terminal devices TAA. Then, for example, the private branch exchange CX is connected to external telephones ICT (TL and TAB) such as fixed telephones TL (TL-1 to TL-3) and mobile telephones TAB (TAB-1 and TAB-2) via a public telephone network PN such as a fixed telephone network and a mobile telephone network, and controls outside calls such as outgoing calls, incoming calls, and talks between the external telephone ICT (TL or TAB) and the mobile terminal device TAA to conduct the outside calls between the external telephone ICT (TL or TAB) and the mobile terminal device TAA. The private branch exchange CX is, for example, a digital exchange or an Internet protocol private branch exchange (IP-PBX). The external telephone ICT includes one or a plurality of fixed telephones TL and one or a plurality of mobile telephones TAB handled by the medical personnel, and further includes, as will be described later, one or a plurality of fixed telephones TL and one or a plurality of mobile telephones TAB handled by persons at the third notification destination to which a message of a last moment (near-death or deathbed) of the monitored person Ob is notified. In the example illustrated in Fig. 1, two mobile telephones TAB-1 and TAB-2 are illustrated, and three fixed telephones TL-1 to RL-3 are illustrated. Then, in the example illustrated in Fig. 1, each of these two mobile telephones TAB-1 and TAB-2 is handled by each person of the medical personnel and each of these three fixed telephones TL-1 to TL-3 is handled by each person at the third notification destination.

The sensor device SU has a communication function to communicate with other devices SV, SP, and TAA via the network NW, and the like, and detects a predetermined event regarding the monitored person Ob and notifies the management server device SV of the detected event, performs a voice call with the terminal devices SP and TAA, and generates an image including a moving image and distributes the moving image to the terminal devices SP and TAA. The sensor device SU is arranged in association with each monitored person Ob. The predetermined event (phenomenon) favorably includes an event requiring handling (deal). In the present embodiment, the predetermined event includes a preset predetermined action in the monitored person Ob, includes respiratory abnormality, and includes a nurse call. Such a sensor device SU includes, for example, as illustrated in Fig. 2, a sensor unit 11, a sensor-side sound input/output unit (SU sound input/output unit) 12, a nurse call reception operation unit 13, a sensor-side control processing unit (SU control processing unit) 14, a sensor-side communication interface unit (SU communication IF unit) 15, and a sensor-side storage unit (SU storage unit) 16.

The sensor unit 11 is a device connected to the SU control processing unit 14 and which detects preset predetermined amounts in the monitored person Ob according to the control of the SU control processing unit 14. The predetermined amounts are appropriately determined according to monitoring items for the monitored person Ob. In the present embodiment, the monitoring items are the preset predetermined actions and respiratory abnormality, and the predetermined actions are four actions including wake up in which the monitored person Ob has waken up, bed leaving in which the monitored person Ob has left bedding, rolling off in which the monitored person has rolled off the bedding, and fall in which the monitored person Ob has falls down. The sensor unit 11 includes an imaging unit 111 in order to detect the predetermined actions and a respiration measurement unit 112 in order to detect the respiratory abnormality, for example.

The imaging unit 111 is a device connected to the SU control processing unit 14 and which generates image (image data) according to the control of the SU control processing unit 14. The image includes a still image (still image data) and a moving image (moving image data). The imaging unit 111 is disposed in a space where the monitored person Ob is scheduled to be located (a location space or the room RM in the arrangement place in the example illustrated in Fig. 1) in a monitorable manner, images the location space from above as an object to be imaged, generates an image (image data) that is a bird's-eye view of the object to be imaged, and outputs the image of the object to be imaged (object image) to the SU control processing unit 14. Favorably, since it is highly probable that the entire monitored person Ob can be imaged, the imaging unit 111 is arranged to image the object to be imaged from directly above a preset head-planned position (usually, a pillow arrangement position) where the head of the monitored person Ob is scheduled to be positioned in the bedding (for example, a bed) on which the monitored person Ob lies down. The sensor device SU acquires an image of the monitored person Ob imaged by the imaging unit 111 from above the monitored person Ob, favorably an image of the monitored person Ob imaged from directly above the head-planned position. The sensor device SU outputs the generated image (object image) of the monitored person Ob to the SU control processing unit 14.

Such an imaging unit 111 may be a device that generates a visible light image. However, in the present embodiment, the imaging unit 111 is a device that generates an infrared image to enable monitoring of the monitored person Ob even in a relatively dark place. The imaging unit 111 is, in the present embodiment, a digital infrared camera including an imaging optical system that forms an infrared optical image of the object to be imaged on a predetermined image plane, an image sensor disposed with a light receiving surface coincident with the image plane and which converts the infrared optical image of the object to be imaged into an electrical signal, an image processing unit that processes an output of the image sensor to generate image data that is data indicating the infrared image of the image sensor, and the like. In the present embodiment, the imaging optical system of the imaging unit 111 is favorably a wide-angle optical system (a so-called wide-angle lens (including a fisheye lens)) having an angle of view capable of imaging the entire room RM.

The respiration measurement unit 112 is a device connected to the SU control processing unit 14 and which measures the respiration of the monitored person Ob according to the control of the SU control processing unit 14. In the present embodiment, measurement of the respiration is indirectly performed in a non-contact manner by measuring the movement of a body surface of chest associated with the respiration. For example, the respiration measurement unit 112 includes a Doppler sensor. The Doppler sensor is a body motion sensor that transmits a transmission wave, receives a reflection wave of the transmission wave reflected by an object, and outputs a Doppler signal of a Doppler frequency component on the basis of the transmission wave and the reflection wave. In a case where the object is moving, the frequency of the reflection wave is shifted in proportion to a moving speed of the object due to the so-called Doppler effect, and therefore a difference (Doppler frequency component) is generated between the frequency of the transmission wave and the frequency of the reflection wave. The Doppler sensor generates a signal of the Doppler frequency component as the Doppler signal (a measurement result of the respiration measurement unit 112), and outputs the Doppler signal to the SU control processing unit 14. The transmission wave may be an ultrasonic wave, a microwave, or the like. In the present embodiment, the transmission wave is a microwave. Since the microwave is transmitted through clothing and reflected at the body surface of the monitored person Ob, even if the monitored person Ob is wearing clothes, the movement of the body surface of the chest associated with the respiration can be detected, which is favorable.

The SU sound input/output unit 12 is a circuit connected to the SU control processing unit 14 and which acquires an external sound and inputs the external sound to the sensor device SU, and is a circuit that generates and outputs a sound according to an electrical signal representing the sound according to the control of the SU control processing unit 14. The SU sound input/output unit 12 includes, for example, a microphone and the like that converts acoustic vibration of the sound into the electrical signal, and a speaker and the like that converts the electrical signal of the sound into acoustic vibration of the sound. The SU sound input/output unit 12 outputs the electrical signal representing the external sound to the SU control processing unit 14, and converts the electrical signal input from the SU control processing unit 14 into the acoustic vibration of the sound and outputs the sound.

The nurse call reception operation unit 13 is a switch circuit connected to the SU control processing unit 14 and is a switch circuit such as a push button-type switch for inputting a nurse call to the sensor device SU. The nurse call reception operation unit 13 may be connected to the SU control processing unit 14 via a wire, or may be connected to the SU control processing unit 14 by short-range wireless communication such as Bluetooth (registered trademark) standard, for example.

The SU communication IF unit 15 is a communication circuit connected to the SU control processing unit 14 and which performs communication according to the control of the SU control processing unit 14. The SU communication IF unit 15 generates a communication signal that contains data to be transferred input from the SU control processing unit 14 according to a communication protocol used in the network NW of the monitored person monitoring system MS, and transmits the generated communication signal to other devices SV, SP, and TAA via the network NW. The SU communication IF unit 15 receives a communication signal from another device SV, SP, or TAA via the network NW, fetches data from the received communication signal, converts the fetched data into data in a format processable by the SU control processing unit 14, and outputs the data to the SU control processing unit 14. The SU communication IF unit 15 includes, for example, a communication interface circuit according to the IEEE 802.11 standard or the like.

The SU storage unit 16 is a circuit connected to the SU control processing unit 14 and which stores various predetermined programs and various predetermined data according to the control of the SU control processing unit 14. The various predetermined programs include, for example, an SU control program for controlling each part of the sensor device SU according to the function of the each part, an action detection processing program for detecting the predetermined action in the monitored person Ob on the basis of the object image of the imaging unit 111 in the sensor unit 11 and notifying the management server apparatus SV of the detected action, a respiratory abnormality determination program for determining whether the respiration of the monitored person is abnormal on the basis of the measurement result of the respiration measurement unit 112 in the sensor section 11 and notifying the management server device SV of the respiratory abnormality, an SU nurse call processing program for notifying the management server device SV of reception of a nurse call when the nurse call reception operation unit 13 has received the nurse call, and performing a voice call with the terminal device SP or TAA using the SU sound input/output unit 12 and the like, and a control processing program such as an SU streaming processing program for streaming the moving image generated by the imaging unit 111 to the terminal device SP or TAA that has requested the moving image. The various predetermined data includes a sensor device identifier (sensor ID) that is an identifier for specifying and identifying its own sensor device SU, and data necessary for executing the programs, such as a communication address of the management server device SV. The SU storage unit 16 includes, for example, a read only memory (ROM) that is a nonvolatile storage element, an electrically erasable programmable read only memory (EEPROM) that is a rewritable nonvolatile storage element, and the like. Then, the SU storage unit 16 includes a random access memory (RAM) that serves as a so-called working memory of the SU control processing unit 14, which stores data and the like generated during the execution of the predetermined programs.

The SU control processing unit 14 is a circuit that controls each part of the sensor device SU according to the function of the each part, detects the predetermined event regarding the monitored person Ob and notifies the management server device SV of the detected event, performs a voice call with the terminal device SP or TAA, and generates an image including a moving image and distributes the moving image to the terminal device SP or TAA. The SU control processing unit 14 includes, for example, a central processing unit (CPU) and peripheral circuits thereof. The SU control processing unit 14 functionally includes a sensor-side control unit (SU control unit) 141, an action detection processing unit 142, a respiratory abnormality determination unit 143, a nurse call processing unit 144, and a sensor-side streaming processing unit (SU streaming processing unit) 145 as the control processing program is executed.

The SU control unit 141 controls each part of the sensor device SU according to the function of the each part, and controls the entire sensor device SU.

The action detection processing unit 142 detects the preset predetermined action in the monitored person Ob on the basis of the object image generated by the imaging unit 111 in the sensor unit 11 and notifies the preset predetermined action to the management server device SV. More specifically, in the present embodiment, the predetermined actions include four actions of wake up, bed leaving, rolling off, and fall, as described above. Then, the action detection processing unit 142 detects the head of the monitored person Ob on the basis of the object image imaged by the imaging unit 111, for example, and detects the wake up, bed leaving, rolling off, and fall of the monitored person Ob on the basis of temporal change in the size of the detected head of the monitored person Ob. More specifically, first, a location area of bedding BD and first to third threshold values Th1 to Th3 are stored in advance in the SU storage unit 16 as one of the various predetermined data. The first threshold value Th1 is a value for discriminating the size of the head at a lying posture and the size of the head at a sitting posture in the location area of the bedding BD. The second threshold value Th2 is a value for discriminating whether the size of the head is the size of the head at a standing posture in the room RM excluding the location area of the bedding BD. The third threshold value Th3 is a value for discriminating whether the size of the head is the size of the head at the lying posture in the room RM excluding the location area of the bedding BD. The action detection processing unit 142 extracts a moving object area as an area of the person of the monitored person Ob from the object image by, for example, a background difference method or a frame difference method. Next, the action detection processing unit 142 extracts a head area of the monitored person Ob from the extracted moving object area by circular or elliptical Hough transform, by pattern matching using a head model prepared beforehand, or by a neural network learned for head detection, for example. Next, the action detection processing unit 142 detects the wake up, bed leaving, rolling off, and fall from the position and size of the extracted head. For example, in a case where the position of the extracted head is within the location area of the bedding BD and the size of the extracted head is temporally changed from the size at the lying posture to the size at the sitting posture by using the first threshold value Th1, the action detection processing unit 142 determines that the action is the wake up and detects the wake up. For example, in a case where the position of the extracted head is temporally changed from within the location area of the bedding BD to the outside of the location area of the bedding BD, and the size of the extracted head is temporally changed from a certain size to the size at the standing position by using the second threshold value Th2, the action detection processing unit 142 determines that the action is the bed leaving and detects the bed leaving. For example, in a case where the position of the extracted head is temporally changed from within the location area of the bedding BD to the outside of the location area of the bedding BD, and the size of the extracted head is temporally changed from a certain size to the size at the lying posture by using the third threshold value Th3, the action detection processing unit 142 determines the rolling off and detects the rolling off. For example, in a case where the position of the extracted head is within the room RM excluding the location area of the bedding BD, and the size of the extracted head is temporally changed from a certain size to the size at the lying posture by using the third threshold value Th3, the action detection processing unit 142 determines the fall and detects the fall.

When detecting the predetermined action in this manner, the action detection processing unit 142 notifies a first event notification communication signal that accommodates event information (phenomenon information) representing the content of the predetermined event (phenomenon) regarding the monitored person Ob and notifies the event to the management server device SV by the SU communication IF unit 15. More specifically, the action detection processing unit 142 transmits its own sensor ID, the event information representing the content of the event, and the communication signal (first event notification communication signal) that accommodates an object image regarding the event to the management server device SV via the SU communication IF unit 15. The event information is one or a plurality of the wake up, bed leaving, rolling off, fall, respiratory abnormality, and nurse call (NC) in the present embodiment. Here, the action detection processing unit 142 accommodates one or a plurality of the detected wake up, bed leaving, rolling off, and fall as the event information in the first event notification communication signal. Here, the object image regarding the event is, for example, the image used for detection of the predetermined action. The object image may be at least one of a still image and a moving image. In the present embodiment, a still image is notified first, and a moving image is distributed in response to a request of a user, as will be described below. Note that the moving image may be distributed first, or the still image and the moving image may be transmitted and displayed on the terminal device SP or TAA by screen division.

The respiratory abnormality determination unit 143 determines whether the respiration of the monitored person is abnormal on the basis of a measurement result of the respiration measurement unit 112 in the sensor unit 11 and notifies the management server device SV of the respiratory abnormality. The respiratory abnormality is respiration indicating an abnormal waveform pattern with respect to the normal respiration of the waveform pattern illustrated in Fig. 3A, and includes, for example, the respiratory arrest (Fig. 3B), the sleep apnea (Fig. 3C), the slow respiration (Fig. 3D), the tachypnea (Fig. 3E), the hypopnea (Fig. 3F), the hyperpnea (Fig. 3G), the Kussmaul respiration (Fig. 3H), and the Cheyne-Stokes respiration (Fig. 3I) in the present embodiment. The respiratory arrest is a case where the respiration is stopped for 30 seconds. The sleep apnea is a case where the respiratory arrest for 10 seconds or longer is observed 5 times or more per hour during sleep. The slow respiration is a case where the respiratory rate per minute is 12 or less. The tachypnea is a case where the respiratory rate per minute is 24 or more. The hypopnea is a case where the amplitude of the body motion is smaller than usual (for example, the amplitude is detected as 40% fall of an average value of past one month) in a state where the respiration is shallow. The hyperpnea is a case where the amplitude of the body motion is larger than usual (for example, the amplitude is detected as 40% rise of the average value of the past one month) in a state where the respiration is deep. The Kussmaul respiration is a case in which the respiratory rate per minute is 12 or less, and the amplitude of the body motion is larger than usual (for example, the amplitude is detected by 40% or more larger than the average value of the past one month). The Cheyne-Stokes respiration is a case in which the amplitude of the body motion is increased (for example, the maximum value becomes 1.5 times) and is then decreased every time, and then a state in which an apnea state continues for a predetermined time (for example, 20 seconds to 1 minute) is repeated. Each of these respiratory arrest, sleep apnea, slow respiration, tachypnea, hypopnea, hyperpnea, Kussmaul respiration, and Cheyne-Stokes respiration is detected by a known conventional method on the basis of the measurement result (the Doppler signal of the Doppler sensor in the present embodiment) of the respiration measurement unit 1 12.

When detecting the respiratory abnormality, the respiratory abnormality determination unit 143 notifies the management server device SV to the first event notification communication signal that accommodates the respiratory abnormality as another example of the predetermined event. More specifically, the respiratory abnormality determination unit 143 transmits the first event notification communication signal that accommodates its own sensor ID, the respiratory abnormality as the event information, and an object image regarding the event to the management server device SV via the SU communication IF unit 15. The object image regarding the event is here an image generated by the imaging unit 111 of the sensor unit 1 1 when the respiratory abnormality is detected.

The nurse call processing unit 144 notifies, when the nurse call reception operation unit 13 receives a nurse call, the first event notification communication signal that accommodates the reception of the nurse call as another example of the predetermined event to the management server device SV, and performs a voice call with the terminal device SP or TAA by using the SU sound input/output unit 12 and the like. More specifically, when the nurse call reception operation unit 13 is operated, the nurse call processing unit 144 transmits the first event notification communication signal that accommodates its own sensor ID and the nurse call as the event information to the management server device SV via the SU communication IF unit 15. Note that the first event notification communication signal that accommodates the nurse call as the event information may further accommodate an image generated by the imaging unit 111 of the sensor unit 11 when the nurse call reception operation unit 13 receives the nurse call, as an object image regarding the event. Then, the nurse call processing unit 144 performs a voice call with the terminal device SP or TAA by, for example, a voice over Internet protocol (VoIP), using the SU sound input/output unit 12 and the like.

The SU streaming processing unit 145 distributes, in a case where the fixed terminal device SP or the mobile terminal device TAA requests distribution of a moving image via a communication IF unit 3, a moving image (for example, a live moving image) generated by the imaging unit 111 to the fixed terminal device SP or the mobile terminal device TAA that has requested the distribution via the SU communication IF unit 15 by streaming reproduction.

In Fig. 1, as an example, four; first to fourth sensor devices SU-1 to SU-4 are illustrated, and the first sensor device SU-1 is disposed in a room RM-1 (not illustrated) of a person A Ob-1 who is one of the monitored persons Ob, the second sensor device SU-2 is disposed in a room RM-2 (not illustrated) of a person B Ob-2 who is one of the monitored persons Ob, the third sensor device SU-3 is disposed in a room RM-3 (not illustrated) of a person C Ob-3 who is one of the monitored persons Ob, and the fourth sensor device SU-4 is disposed in a room RM-4 (not illustrated) of a person D Ob-4 who is one of the monitored persons Ob.

The management server device SV has a communication function to communicate with other devices SU, TAA, and SP via the network NW. When receiving the notification of the predetermined event from the sensor device SU, the management server device SV manages information regarding monitoring the monitored person Ob (monitoring information (in the present embodiment, the predetermined events (the type of the predetermined action and the respiratory abnormality detected by the sensor device SU and the nurse call received by the sensor device SU), images of the monitored person Ob (the still image and the moving image), and the time when the notification has been received, and the like)), notifies (re-notifies, re-informs, or transmits) the predetermined terminal device SP or TAA of the predetermined event, provides data according to the request of the client (the terminal device SP, TAA, or the like in the present embodiment) to the client, and manages the entire monitored person monitoring system MS. Then, in the present embodiment, the management server device SV further communicates with the external telephone ICT (TL or TAB) via the network NW and the public telephone network PN, notifies a predetermined external telephone ICT of the determination result of the respiratory abnormality determination unit 143 in a case where a predetermined notification condition is satisfied or when receiving an instruction from the terminal device TAA, and notifies the predetermined external telephone ICT associated with the monitored person Ob of the message indicating that the monitored person Ob is at the last moment when receiving an instruction from the external telephone ICT. Such a management server device SV includes, for example, as illustrated in Fig. 4, a server-side communication interface unit (SV communication IF unit) 21, a server-side control processing unit (SV control processing unit) 22, and a server-side storage unit (SV storage unit) 23.

The SV communication IF unit 21 is a communication circuit connected to the SV control processing unit 22 and which performs communication according to the control of the SV control processing unit 22, similarly to the SU communication IF unit 15. The SV communication IF unit 21 includes, for example, a communication interface circuit according to the IEEE 802.11 standard or the like.

The SV storage unit 23 is a circuit connected to the SV control processing unit 22 and which stores various predetermined programs and various predetermined data according to the control of the SV control processing unit 22. The various predetermined programs include, for example, an SV control program for controlling each part of the management server device SV according to the function of the each part, an SV monitoring notification processing program for executing predetermined information processing regarding monitoring the monitored person Ob and notifying predetermined information as needed, and a control processing program such as a clock program for performing timing. The SV monitoring notification processing program includes an SV monitoring processing program for executing predetermined information processing regarding monitoring the monitored person Ob, a notification processing program for notifying the predetermined information as needed, and the like. The various predetermined data includes its own server identifier (server ID) that is an identifier for specifying and identifying the management server device SV, the monitoring information of the monitored person Ob, inter-device information indicating information among the devices SU, SP, TAA, and ICT (TL and TAB) such as notification destinations of the predetermined event, sensor information regarding the sensor device SU, data necessary for execution of programs such as symptom history information indicating a history of a symptom of the monitored person Ob, and the like. To store the monitoring information, the inter-device information, the sensor information, and the symptom history information, respectively, the SV storage unit 23 functionally includes a server-side monitoring information storage unit (SV monitoring information storage unit) 231, an inter-device information storage unit 232, a server-side sensor information storage unit (SV sensor information storage unit) 233, and a symptom history information storage unit 234.

The SV monitoring information storage unit 231 stores the monitoring information of the monitored person Ob transmitted/received to/from each device SU, SP, TAA, or TAB. More specifically, in the present embodiment, the SV monitoring information storage unit 231 stores, as the monitoring information, the sensor ID, the event information (the wake up, bed leaving, rolling off, fall, respiratory abnormality, and nurse call in the present embodiment), a reception time, object images (a still image and a moving image), and presence or absence of handling/resume in association with one another on the basis of each piece of information accommodated in communication signals such as the first event notification communication signal, and a deal reception notification communication signal, and a resume reception notification communication signal to be described below.

In the present embodiment, the monitoring information is stored in the SV monitoring information storage unit 321 in a table format. A server-side monitoring information table (SV monitoring information table) MT-SV for registering the monitoring information includes, for example, as illustrated in Fig. 5, a sensor ID field 2311 for registering the sensor ID accommodated in the communication signal received from each device SU, SP, or TAA, an event field (event field) 2312 for registering the event information accommodated in the received communication signal, a reception time field 2313 for registering the reception time of the received communication signal, a still image field 2314 for registering the still image accommodated in the received communication signal, a moving image field 2315 for registering the communication address (for example, an IP address) of the sensor device SU corresponding to the sensor ID accommodated in the received communication signal, as an acquisition source of a live moving image, a deal/resume field 2316 for registering presence or absence of reception of deal for event information accommodated in the received communication signal and presence or absence of reception of resume to instruct continuation of monitoring of the respiration, and includes a record for each received communication signal (each event). In the still image field 2314, image data of the still image may be registered, for example, and a file name of the image data of the still image may be registered, for example. In the deal/resume field 2316, as will be described below, a flag (deal/resume flag) is registered, which represents whether the terminal device SP or TAA has received an intention to handle (deal/treat/measure) the event information accommodated in the received communication signal ("deal"), and represents whether the terminal device SP or TAA or the external telephone ICT (the mobile telephone TAB in the present embodiment) handled by the medical personnel has received an instruction to continue monitoring of the respiration. For example, in the present embodiment, in the deal/resume field 2316, a deal/resume flag "1" meaning that the terminal device SP or TAA has received an intention ("deal") to handle the types of the predetermined actions and the nurse call accommodated as the event information in the received communication signal (the event information registered in the event field 2312) or a deal/resume flag "0" meaning that the terminal device SP or TAA has not received the intention ("deal") to handle the event information accommodated in the received communication signal is registered. Further, in the deal/resume field 2316, a deal/resume flag "1" meaning that the terminal device SP or TAA or the mobile telephone TAB handled by the medical personnel has received an intention ("resume") to continue the monitoring of the respiration regarding the respiratory abnormality (the event information registered in the event field 2312) accommodated as the event information in the received communication signal, or a deal/resume flag "0" meaning that the terminal device SP or TAA or the mobile telephone TAB has not received the intention ("resume") to continue the monitoring of the respiration for the event information accommodated in the received communication signal is registered. Note that the deal/resume flag "0" meaning non-reception is registered by default in the deal/resume field 2316. Note that, in a case where the first event notification communication signal accommodates a detection time when the predetermined action or the respiratory abnormality has been detected or a nurse call reception time when the nurse call has been received, the detection time or the nurse call reception time may be registered in place of the reception time.

In the present embodiment, the inter-device information storage unit 232 stores, as the inter-device information, correspondence (condition-based notification destination correspondence or condition-based notification destination information) between the sensor IDs as transmission sources and terminal IDs as the notification destinations, indicating a plurality of notification destinations classified according to predetermined criteria, and correspondence (communication address correspondence) between the IDs (the sensor IDs and the terminal IDs) of the devices SU, SP, TAA, TL, and TAB and their communication addresses, with respect to the first event notification communication signal transmitted from the sensor device SU. The terminal ID is a terminal identifier for specifying and identifying the terminal device SP or TAA or the external telephone ICT (TL or TAB).

In the present embodiment, the condition-based notification destination correspondence and the communication address correspondence are respectively stored in the inter-device information storage unit 232 in a table format. A condition-based notification destination correspondence information table AT that registers the condition-based notification destination correspondence includes, for example, as illustrated in Fig. 6A, a sensor ID field 2321 for registering the sensor ID of the sensor device SU of the transmission source (in other words, the monitored person Ob), a notification destination A field 2322 for registering a notification destination A that is a notification destination corresponding to the sensor ID (in other words, the monitored person Ob) registered in the sensor ID field 2321 and of a case where the notification condition to be described below is a condition A, a notification destination B field 2323 for registering a notification destination B that is a notification destination corresponding to the sensor ID (in other words, the monitored person Ob) registered in the sensor ID field 2321 and of a case where the notification condition is a condition B, and a notification destination C field 2324 for registering a notification destination C that is a notification destination to which a predetermined message indicating that the monitored person Ob corresponding to the sensor ID registered in the sensor ID field 2321 is at the last moment is notified, and includes a record for each sensor ID (sensor device SU). As described above, since the sensor device SU is provided in association with the monitored person Ob for each of monitored persons Ob, the sensor ID means the monitored person Ob. In the present embodiment, the notification condition includes the condition A (normal or the degree of urgency is small) that does not require determination by the medical personnel as to whether the monitored person Ob is at the last moment and is a case where the degree of urgency is relatively low, and the condition B (at emergency or the degree of urgency is large) that requires the determination by the medical personnel as to whether the monitored person Ob is at the last moment and is a case where the degree of urgency is relatively high. Therefore, in the notification destination A field 2322, the terminal ID of the terminal device SP or TAA handled by the monitor such as the nurse or the caretaker is registered. In the notification destination B field 2323, the terminal ID of the external telephone ICT (the mobile telephone TAB in the present embodiment) handled by the medical personnel such as a doctor is registered. In the notification destination C field 2324, the terminal ID of the external telephone ICT (the fixed telephone TL in the present embodiment) handled by a close relative such as a family member of the monitored person Ob is registered. Therefore, in the present embodiment, the predetermined criteria includes three criteria: a first criterion as to whether a person is the monitor of the monitored person Ob; a second criterion as to whether a person can determine the last moment of the monitored person Ob; and a third criterion as to whether a person requires contact at the last moment of the monitored person Ob, and a person who satisfies the criterion in each criterion is the notification destination.

A communication address correspondence information table DT for registering the communication address correspondence includes, for example, as illustrated in Fig. 6B, a terminal ID field 2325 for registering the terminal ID of the terminal device SP or TAA or the external telephone ICT (TL or TAB), and a communication address field 2326 for registering the communication address of the terminal device SP or TAA or the external telephone ICT (TL or TAB) corresponding to the terminal ID registered in the terminal ID field 2325, and includes a record for each terminal ID (each device SP, TAA, TL, or TAB).

Note that each of the sensor ID, the server ID, and the terminal ID may be, for example, a serial number composed of a predetermined symbol string, or may be a communication address (in this case, the communication address correspondence can be omitted). Further, in the case where the external telephone ICT is the fixed telephone TL, a telephone number may be registered in the communication address field 2326.

The SV sensor information storage unit 233 stores the sensor information. In the present embodiment, the sensor information is information regarding the sensor device SU and is information in which the sensor ID of the sensor device SU and a monitored person name of the monitored person Ob are associated with each other.

In the present embodiment, such sensor information is stored in the SV sensor information storage unit 233 in a table format. More specifically, a sensor information table ST-SV for registering the sensor information includes, for example, as illustrated in Fig. 7, a sensor ID field 2331 for registering the sensor ID, an arrangement place field 2332 for registering an arrangement place of the sensor device SU having the sensor ID registered in the sensor ID field 2331, a monitored person name field 2333 for registering the monitored person name of the monitored person Ob monitored by the sensor device SU having the sensor ID registered in the sensor ID field 2331 (that is, the monitored person Ob located in the arrangement place of the sensor device SU having the sensor ID registered in the sensor ID field 2331), and a remark field 2334 for registering remarks regarding the sensor device SU having the sensor ID registered in the sensor ID field 2331, the arrangement place, and the monitored person Ob, and includes a record for each sensor ID (that is, each sensor device SU, still in other words, the monitored person Ob).

The symptom history information storage unit 234 stores a symptom history as symptom history information in association with the sensor ID (that is, the sensor device SU, still in other words, the monitored person Ob). The symptoms may be arbitrary, and the symptom history information may include a past history of heart failure.

The SV control processing unit 22 is a circuit for controlling each part of the management server device SV according to the function of the each part, and when receiving notification of the predetermined event from the sensor device SU, manages the monitoring information regarding monitoring of the monitored person Ob, notifies (re-notifies, re-informs, or transmits) the predetermined event to the predetermined terminal device SP or TAA, provides the data in response to the client request to the client, and manages the entire monitored person monitoring system MS. Then, in the present embodiment, the SV control processing unit 22 further notifies a predetermined external telephone ICT of the determination result of the respiratory abnormality determination unit 143 in a case where a predetermined notification condition is satisfied or when receiving an instruction from the terminal device TAA, and notifies the predetermined external telephone ICT associated with the monitored person Ob of the message indicating that the monitored person Ob is at the last moment when receiving an instruction from the external telephone ICT. The SV control processing unit 22 includes, for example, a CPU and peripheral circuits thereof. The SV control processing unit 22 functionally includes a server-side control unit (SV control unit) 221, a server-side monitoring notification processing unit (SV monitoring notification processing unit) 222, and a clock unit 223 as the control processing program is executed.

The SV control unit 221 controls each part of the management server device SV according to the function of the each part to control the entire management server device SV.

The SV monitoring notification processing unit 222 executes predetermined information processing regarding monitoring of the monitored person Ob and notifies predetermined information as needed, and functionally includes an SV monitoring processing unit 2221 and a notification processing unit 2222 in the present embodiment.

When receiving notification of events regarding detection of the predetermined action and the nurse call from the sensor device SU, the SV monitoring processing unit 2221 manages the monitoring information regarding monitoring of the monitored person Ob and notifies the predetermined terminal device SP or TAA of the predetermined events. More specifically, when receiving the first event notification communication signal regarding the detection of the predetermined action and the nurse call from the sensor device SU, the SV monitoring processing unit 2221 stores (records) the monitoring information regarding monitoring of the monitored person Ob accommodated in the received first event notification communication signal to the SV monitoring information storage unit 231. Then, the SV monitoring processing unit 2221 selects (searches for) the notification destination corresponding to the sensor device SU that has transmitted the received first event notification communication signal from the notification destination A field 2322 in the condition-based notification destination correspondence information table AT stored in the inter-device information storage unit 232, and transmits a second event notification communication signal to the selected terminal device SP or TAA. This selection (search processing) is performed on the basis of the sensor ID corresponding to the sensor device SU that has transmitted the received first event notification communication signal. In the case where the event information accommodated in the first event notification communication signal is the predetermined action (one or a plurality of the wake up, bed leaving, rolling off, and fall), the second event notification communication signal accommodates the sensor ID accommodated in the first event notification communication signal, the event information and the object images, and the communication address corresponding to the sensor device SU having the sensor ID accommodated in the first event notification communication signal, as a downloading destination of the moving image. This communication address is selected (searched) from the communication address correspondence on the basis of the sensor ID corresponding to the sensor device SU that has transmitted the received first event notification communication signal. In the case where the event information accommodated in the first event notification communication signal is the nurse call, the second event notification communication signal accommodates the sensor ID and the event information (nurse call) accommodated in the first event notification communication signal.

When receiving notification of the event regarding detection of the respiratory abnormality from the sensor device SU, the notification processing unit 2222 manages the monitoring information regarding monitoring of the monitored person Ob, obtains a notification condition on the basis of the respiratory abnormality, selects a notification destination corresponding to the obtained notification condition as a first notification destination from the condition-based notification destination correspondence registered in the condition-based notification destination information table AT stored in the inter-device information storage unit 232, and notifies the selected first notification destination of the respiratory abnormality. More specifically, when receiving the first event notification communication signal regarding the detection of the respiratory abnormality from the sensor device SU, the notification processing unit 2222 stores (records) the monitoring information regarding monitoring of the monitored person Ob accommodated in the received first event notification communication signal to the SV monitoring information storage unit 231, similarly to the SV monitoring processing unit 2221. Then, the notification processing unit 2222 obtains the notification condition on the basis of the respiratory abnormality accommodated as the event information in the received first event notification communication signal. In the present embodiment, when obtaining the notification condition, the notification processing unit 2222 obtains the notification condition further on the basis of the symptom history information stored in the symptom history information storage unit 234 and associated with the monitored person Ob corresponding to the sensor ID accommodated in the received first event notification communication signal. More specifically, in a case of the respiratory abnormality and a case where the symptom history information of the monitored person Ob includes the past history of heart failure, the notification processing unit 2222 determines the condition B that is the case where the degree of urgency is relatively high as the notification condition, selects (searches for) the first notification destination corresponding to the sensor device SU that has transmitted the received first notification destination from the notification destination B field 2323 of the condition-based notification destination correspondence information table AT stored in the inter-device information storage unit 232 with the notification destination B as the first notification destination, and transmits a communication signal (near-death determination request communication signal) for requesting determination as to whether the monitored person Ob is at the last moment to the selected external telephone ICT (the mobile telephone TAB handled by the medical personnel in the present embodiment). This selection (search processing) is performed on the basis of the sensor ID corresponding to the sensor device SU that has transmitted the received first event notification communication signal. The near-death determination request communication signal accommodates the name of the monitored person Ob corresponding to the sensor ID accommodated in the first event notification communication signal, the sensor ID accommodated in the first event notification communication signal, the event information (respiratory abnormality) and the object images regarding the event information, and a predetermined message (for example, a "near-death determination request") requesting the determination as to whether the monitored person Ob is at the last moment. Note that, in the present embodiment, to perform a so-called wait-and-see operation to see the progress of the condition of the monitored person Ob from previous transmission of the near-death determination request communication signal, retransmission of the near-death determination request communication signal is executed after a lapse of a preset predetermined time from the previous transmission of the near-death determination request communication signal. The predetermined time is arbitrarily appropriately set and is set to 3 hours or 6 hours, for example. To take account of the condition of the monitored person Ob, the predetermined time may be set via the mobile telephone TAB by the medical personnel (for example, a doctor) who handles the mobile telephone TAB that has received the near-death determination request communication signal. Meanwhile, in a case of the respiratory abnormality and a case where the symptom history information of the monitored person Ob is another case excluding the case of the past history of heart failure, the notification processing unit 2222 determines the condition A that is the case where the degree of urgency is relatively low as the notification condition, selects (searches for) the first notification destination corresponding to the sensor device SU that has transmitted the received first notification destination from the notification destination A field 2322 of the condition-based notification destination correspondence information table AT stored in the inter-device information storage unit 232 with the notification destination A as the first notification destination, and transmits the selected terminal device SP or TAA of the second event notification communication signal. The second event notification communication signal accommodates the sensor ID accommodated in the first event notification communication signal, the event information (respiratory abnormality), and the object images regarding the event information.

Then, in a case of receiving an instruction to forward the respiratory abnormality from the terminal device SP or TAA as the first notification destination to which the respiratory abnormality has been notified with the second event notification communication signal, the notification processing unit 2222 performs re-notification processing of selecting another notification destination excluding the notification destination to which the respiratory abnormality has already been notified as a second notification destination from among the plurality of notification destinations, and notifying the selected second notification destination of the respiratory abnormality. More specifically, in a case of receiving the communication signal (near-death determination desire communication signal) for forwarding the respiratory abnormality from the terminal device SP or TAA as the first notification destination, the notification processing unit 2222 selects (searches for) the second notification destination corresponding to the sensor ID accommodated in the received near-death determination desire communication signal from the notification destination B field 2323 of the condition-based notification destination correspondence information table AT stored in the inter-device information storage unit 232, and transmits the near-death determination request communication signal to the selected external telephone ICT (the mobile telephone TAB handled by the medical personnel in the present embodiment). The near-death determination request communication signal accommodates the name of the monitored person Ob corresponding to the sensor ID accommodated in the near-death determination desire communication signal, the sensor ID accommodated in the near-death determination desire communication signal, the event information (respiratory abnormality) and the object images regarding the event information, and a predetermined message (for example, a "near-death determination request") requesting the determination as to whether the monitored person Ob is at the last moment.

Note that the near-death determination desire communication signal accommodates, as will be described below, the sensor ID accommodated in the second event notification communication signal received by the terminal device SP or TAA as the first notification destination, the event information (respiratory abnormality) and the object images regarding the event information, and an instruction to forward the respiratory abnormality as a desire for the near-death determination.

Further, in a case of receiving a second instruction to notify a message indicating that the monitored person Ob is at the last moment from the external telephone ICT (the mobile telephone TAB handled by the medical personnel in the present embodiment) as the first or second notification destination to which the respiratory abnormality has been notified with the near-death determination request communication signal, the notification processing unit 2222 notifies a third notification associated with the monitored person Ob of the message. More specifically, in a case of receiving a communication signal (desire for near-death notification communication signal) for obtaining notification of the message indicating that the monitored person Ob is at the last moment from the mobile telephone TAB as the first or second notification destination, the notification processing unit 2222 selects (searches for) the notification destination C corresponding to the sensor ID accommodated in the received desire for near-death notification communication signal from the notification destination C field 2324 of the condition-based notification destination correspondence information table AT stored in the inter-device information storage unit 232, and transmits a near-death notification communication signal to the selected external telephone ICT (the fixed telephone TL in the present embodiment). The near-death notification communication signal accommodates the message indicating that the monitored person Ob is at the last moment. The message may be text but the message is favorably a voice so that the message is available by the fixed telephone TL or the mobile telephone.

Note that the desire for near-death notification communication signal accommodates, as will be described below, the sensor ID accommodated in the near-death determination request communication signal and the second instruction notifying the message indicating that the monitored person Ob is at the last moment.

The clock unit 223 measures the hour and minute. The timer unit 223 may further measure the date and time.

Note that the management server device SV may further include, as illustrated by the broken lines in Fig. 4, a server-side input unit (SV input unit) 24 connected to the SV control processing unit 22 and for inputting various commands and various data, a server-side output unit (SV output unit) 25 for outputting the various commands and various data input at the SV input unit 24 and the monitoring information regarding monitoring of the monitored person Ob, a server-side interface unit (SVIF unit) 26 for inputting and outputting data to and from an external device, and the like, as needed.

Such a management server device SV can be constituted by, for example, a computer with a communication function.

The fixed terminal device SP is a device having a communication function to communication with other devices SU, SV, and TAA via the network NW, a display function to display predetermined information, an input function to input predetermined instructions and data, and the like, and which functions as a user interface (UI) of the monitored person monitoring system MS by inputting the predetermined instructions and data to be given to the management server device SV and the mobile terminal device TAA and displaying the monitoring information obtained by the sensor device SU. Such a fixed terminal device SP can be constituted by, for example, a computer with a communication function. Note that the fixed terminal device SP as an example of the terminal device operates similarly to the mobile terminal device TAA. However, in the present specification, the mobile terminal device TAA as another example of the terminal device will be described.

The mobile terminal device TAA is a device having a communication function to communicate with other devices SV, SP, SU via the network NW, a display function to display predetermined information, an input function to input predetermined instructions and data, a call function to perform a voice call, and the like, and the mobile terminal device TAA is a device for inputting the predetermined instructions and data to be given to the management server device SV and the sensor device SU, displaying the monitoring information obtained by the sensor device SU by the notification from the management server device SV, and responding to the nurse call or speaking by a voice call with the sensor device SU. Then, in the present embodiment, when receiving the second event notification communication signal of the respiratory abnormality from the management server device SV, as will be described below, the mobile terminal device TAA receives an input operation by the user (monitor) for desiring the medical personnel to make the near-death determination as to whether the monitored person is at the last moment and notifies the mobile telephone TAB handled by the medical personnel of the input operation via the management server device SV. Such a mobile terminal device TAA includes, for example, as illustrated in Fig. 8, a terminal-side communication interface unit (TA communication IF unit) 31, a terminal-side control processing unit (TA control processing unit) 32, a terminal-side storage unit (TA storage unit) 33, a terminal-side sound input/output unit (TA sound input/output unit) 34, a terminal-side input unit (TA input unit) 35, a terminal-side display unit (TA display unit) 36, and a terminal-side interface unit (TAIF unit) 37 in the present embodiment.

The TA sound input/output unit 34 is a circuit connected to the TA control processing unit 32, and is a circuit for acquiring an external sound and inputting the sound to the mobile terminal device TAA and is a circuit for generating and outputting a sound according to an electrical signal representing a sound according to the control of the TA control processing unit 32, similarly to the SU sound input/output unit 12.

The TA input unit 35 is a circuit connected to the TA control processing unit 32 and is a circuit for receiving and inputting a predetermined operation to the mobile terminal device TAA, and is, for example, a plurality of input switches to which predetermined functions are allocated. The predetermined operation includes various operations necessary for monitoring such as an input operation of an ID for logging in, a request operation and a termination operation of a voice call, a request operation or a termination operation of a live moving image, an input operation to indicate an intention ("deal") to deal with (handling) of lifesaving, nursing, care, or assistance for the monitored person Ob regarding the notified event, a resume input operation to instruct continuation of monitoring the respiration, an input operation to desire the near-death determination, and a request operation and a termination operation of the measurement result of the respiration measurement unit 112 in the sensor device SU. The TA display unit 36 is a circuit connected to the TA control processing unit 32, and which displays, according to the control of the TA control processing unit 32, predetermined operation content input from the TA input unit 35, the monitoring information regarding the monitored person Ob monitored by the monitored person monitoring system MS (for example, the predetermined event (the type of the predetermined action detected by the sensor device SU, the respiratory abnormality detected by the sensor device SU, or the nurse call received by the sensor device SU), the images (the still image and the moving image) of the monitored person Ob, or the time when the notification has been received), or the like, and is a display device such as a liquid crystal display (LCD) or an organic EL display. In the present embodiment, the TA input unit 35 and the TA display unit 36 constitute a touch panel. In this case, the TA input unit 35 is a position input device that detects and inputs an operation position, such as a resistive film-type or a capacitive-type position input device. The touch panel is provided with the position input device on a display surface of the TA display unit 36, and one or a plurality of input content candidates inputtable to the TA display unit 36 is displayed. When the user (monitor) such as the nurse or the caretaker touches a display position where the input content to be input is displayed, the position is detected by the position input device, and the display content displayed at the detected position is input to the mobile terminal device TAA as operation input content of the user.

The TAIF unit 37 is a circuit connected to the TA control processing unit 32 and which inputs/outputs data to/from an external device according to the control of the TA control processing unit 32. The TAIF unit 37 is, for example, an interface circuit using the Bluetooth (registered trademark) standard, an interface circuit that performs infrared communication such as an IrDA standard, or an interface circuit using a USB standard.

The TA communication IF unit 31 is a communication circuit connected to the TA control processing unit 32 and which performs communication according to the control of the TA control processing unit 32, similarly to the SU communication IF unit 15. The TA communication IF unit 31 includes, for example, a communication interface circuit according to the IEEE 802.11 standard or the like.

The TA storage unit 33 is a circuit connected to the TA control processing unit 32 and which stores various predetermined programs and various predetermined data according to the control of the TA control processing unit 32. The various predetermined programs include, for example, control processing programs such as a TA control program for controlling each part of the mobile terminal device TAA according to the function of the each part, a TA monitoring processing program for executing predetermined information processing regarding monitoring of the monitored person Ob, a call processing program for performing a voice call with the sensor device SU by using the TA sound input/output unit 34 and the like, and a TA streaming processing program for receiving distribution of a moving image from the sensor device SU and displaying the distributed moving image on the TA display unit 36 by streaming reproduction. The various predetermined data includes data necessary for executing the programs such as its own terminal ID, screen information displayed on the TA display unit 36, the monitoring information of the monitored person Ob, and the sensor information regarding the sensor device SU. The TA storage unit 33 includes a ROM and an EEPROM, for example. The TA storage unit 33 includes a RAM serving as a so-called working memory of the TA control processing unit 32, which stores data caused during execution of the predetermined program and the like. To store the monitoring information and the sensor information, respectively, the TA storage unit 33 functionally includes a terminal-side monitoring information storage unit (TA monitoring information storage unit) 331 and a terminal-side sensor information storage unit (TA sensor information storage unit) 332.

The TA monitoring information storage unit 331 stores the monitoring information. In the present embodiment, the TA monitoring information storage unit 331 stores, as the monitoring information, the sensor ID accommodated in the second event notification communication signal received from the management server device SV, the event information (the wake up, bed leaving, rolling off, fall, respiratory abnormality, and nurse call in the present embodiment), the images and the communication address of the sensor device SU at the downloading source of the moving image, the reception time of the second event notification communication signal, the presence or absence of deal, the presence or absence of resume, and the like in association with one another. More specifically, as illustrated in Fig. 5, the TA monitoring information storage unit 331 stores the monitoring information in a terminal-side monitoring information table (TA monitoring information table) MT-TA, similarly to the SV monitoring information table MT-SV.

The TA sensor information storage unit 332 stores the sensor information. The TA sensor information storage unit 332 stores the sensor ID, the arrangement place, the monitored person name, and remarks in association with one another. More specifically, as illustrated in Fig. 7, the TA sensor information storage unit 332 stores the sensor information in a terminal-side sensor information table (TA sensor information table) ST-TA, similarly to the SV sensor information table ST-SV.

The TA control processing unit 32 is a circuit for controlling each part of the mobile terminal devices TAA according to the function of the each part, receiving and displaying the monitoring information of the monitored person Ob, and responding to the nurse call and speaking. Then, in the present embodiment, when receiving the second event notification communication signal of the respiratory abnormality from the management server device SV, the TA control processing unit 32 receives an input operation by the user (monitor) for desiring the medical personnel to make the near-death determination as to whether the monitored person is at the last moment and notifies the mobile telephone TAB handled by the medical personnel of the input operation via the management server device SV. The TA control processing unit 32 includes, for example, a CPU and peripheral circuits thereof. The TA control processing unit 32 functionally includes a terminal-side control unit (TA control unit) 321, a terminal-side monitoring processing unit (TA monitoring processing unit) 322, a call processing unit 323, and a terminal-side streaming processing unit (TA streaming processing unit) 324 as the control processing program is executed.

The TA control unit 321 controls each part of the mobile terminal device TAA according to the function of the each part to control the entire mobile terminal device TAA.

The TA monitoring processing unit 322 executes predetermined information processing regarding monitoring of the monitored person Ob. More specifically, in a case of receiving the second event notification communication signal transmitted by the management server device SV due to the first event notification communication signal transmitted by the sensor device SU, the TA monitoring processing unit 322 stores (records) the monitoring information of the monitored person Ob to the TA monitoring information storage unit 331 on the basis of each piece of the information (data) accommodated in the received second event notification communication signal. The TA monitoring processing unit 322 displays a screen corresponding to each piece of information accommodated in the received second event notification communication signal on the TA display unit 36. Then, when receiving a predetermined input operation from the TA input unit 35, the TA monitoring processing unit 322 executes predetermined processing according to the input operation.

The call processing unit 323 performs a voice call with the sensor device SU by using the TA sound input/output unit 34 and the like. More specifically, for example, the call processing unit 323 uses the TA sound input/output unit 34 and the like and performs a voice call by VoIP with the sensor device SU that is the transmission source having transmitted the first event notification communication signal to the management server device SV, the first event notification communication signal being the cause of the transmission of the second event notification communication signal.

The TA streaming processing unit 324 receives distribution of a moving image from the sensor device SU and displays the distributed moving image on the TA display unit 36 by streaming reproduction.

Such a mobile terminal device TAA can be constituted by a mobile communication terminal device such as a so-called tablet-type computer, a smartphone, or a mobile telephone.

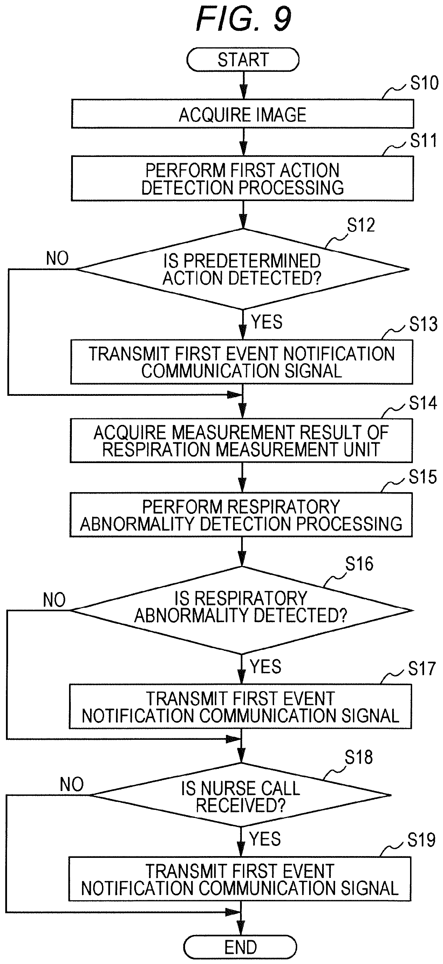

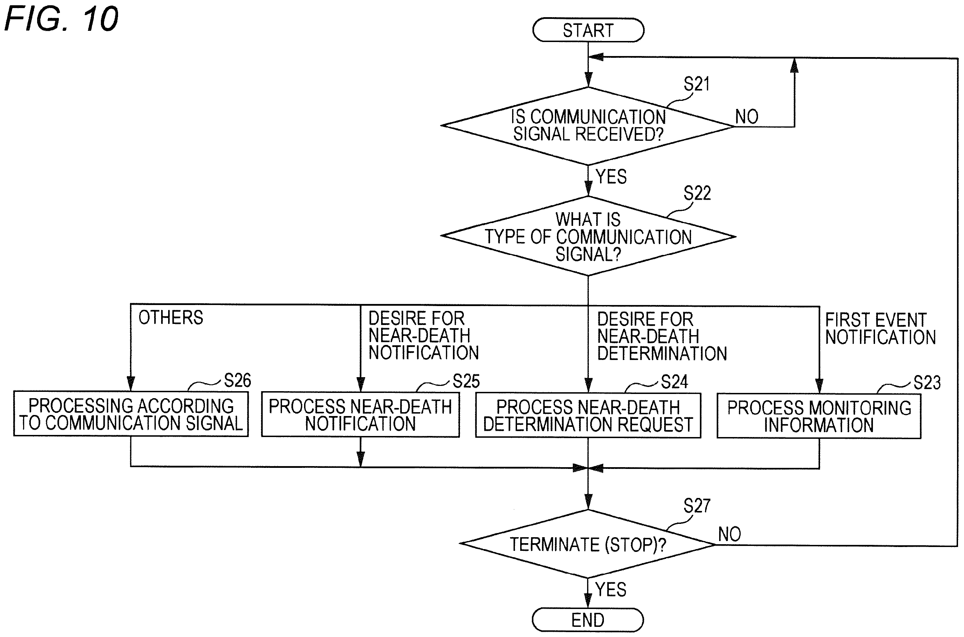

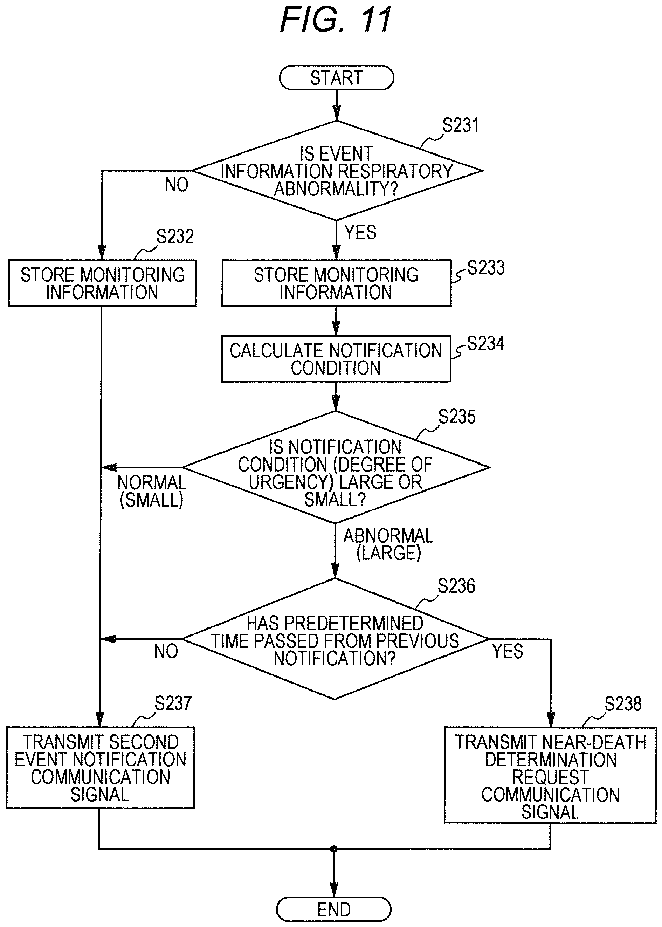

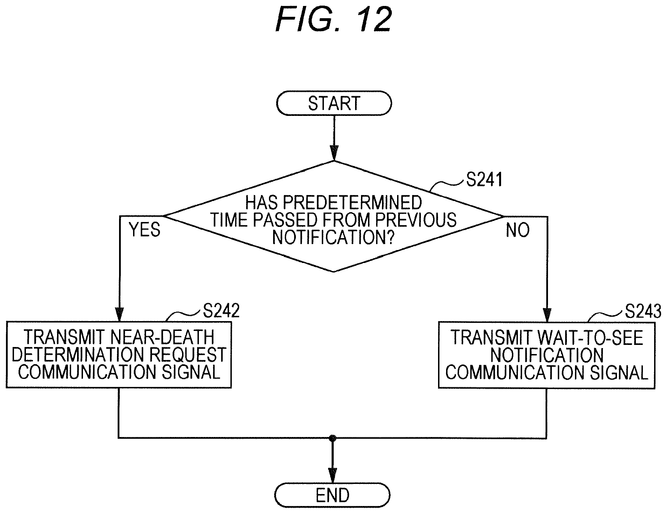

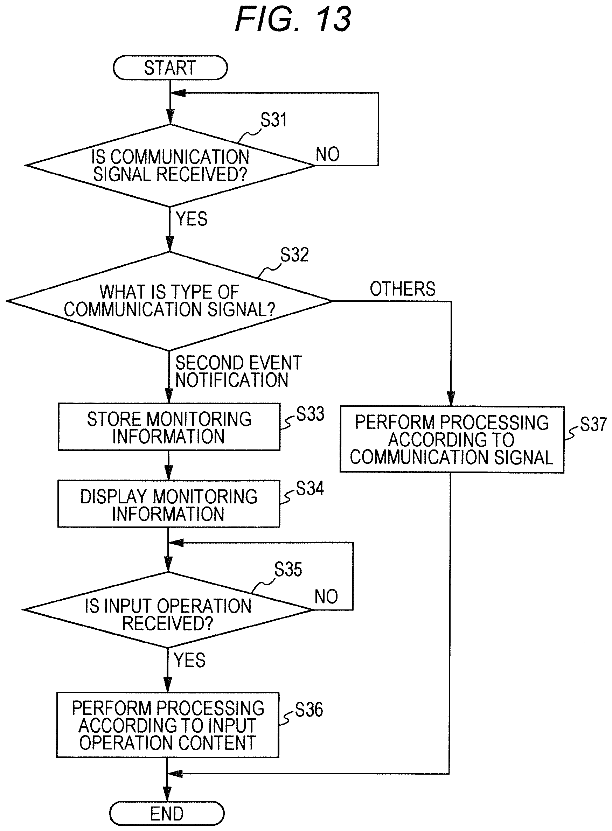

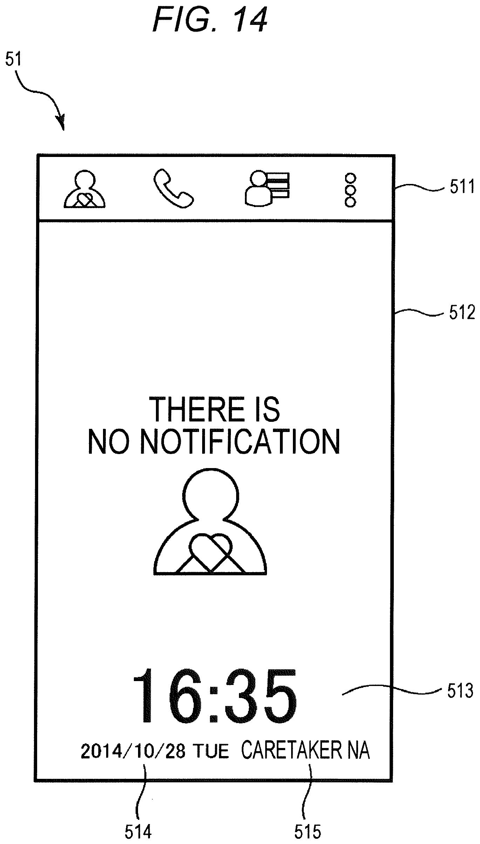

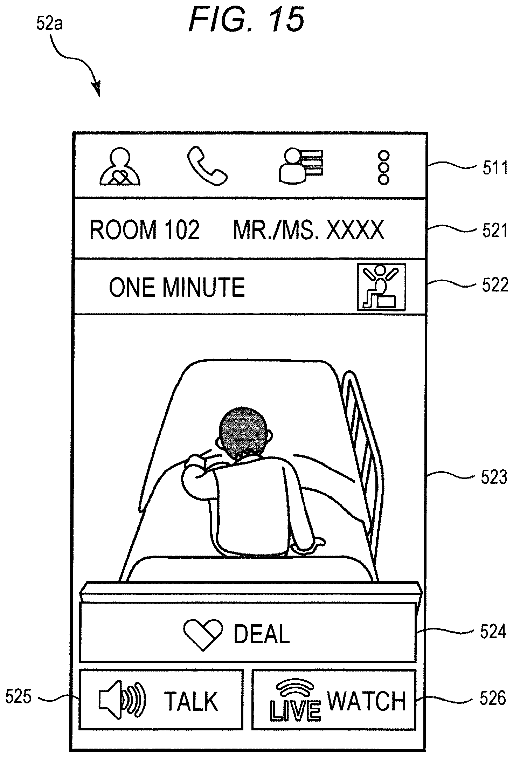

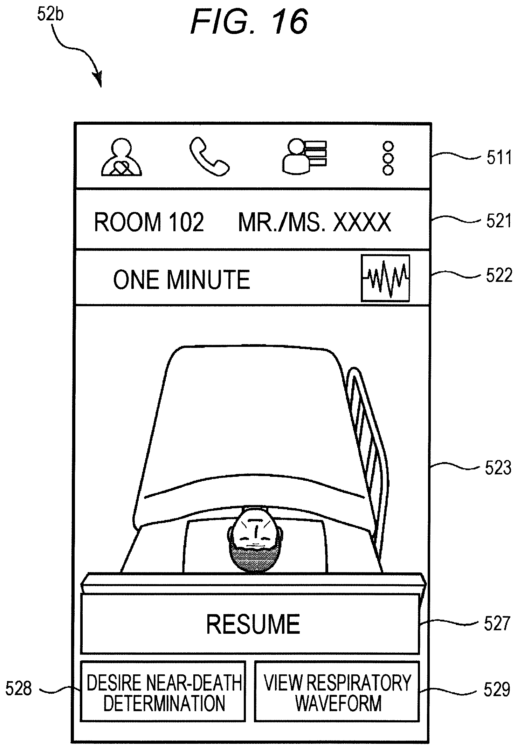

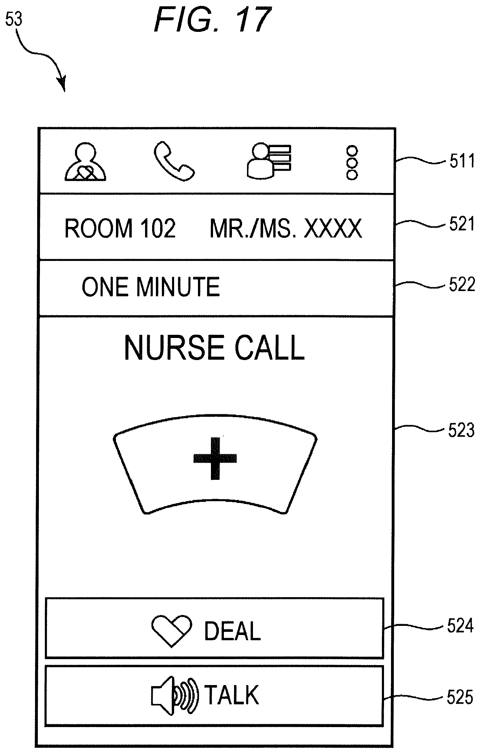

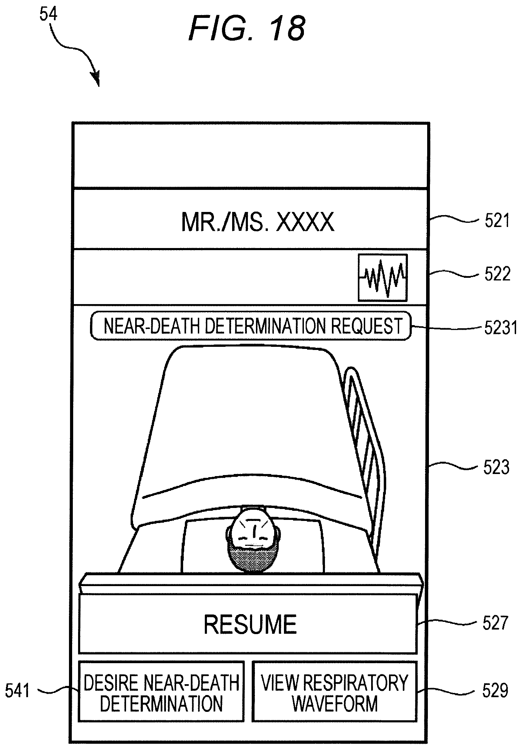

Next, an operation of the present embodiment will be described. Fig. 9 is a flowchart illustrating an operation of the sensor device illustrated in Fig. 2. Fig. 10 is a flowchart illustrating an operation of the management server device illustrated in Fig. 4. Fig. 11 is a flowchart illustrating monitoring information processing in the flowchart illustrated in Fig. 10. Fig. 12 is a flowchart illustrating near-death determination request processing in the flowchart illustrated in Fig. 10. Fig. 13 is a flowchart illustrating an operation of the mobile terminal device illustrated in Fig. 8. Fig. 14 is a diagram illustrating an example of a standby screen displayed on the mobile terminal device illustrated in Fig. 8. Fig. 15 is a diagram illustrating an example of a monitoring information screen in first mode displayed on the mobile terminal device illustrated in Fig. 8. Fig. 16 is a diagram illustrating an example of the monitoring information screen in second mode displayed on the mobile terminal device illustrated in Fig. 8. Fig. 17 is a diagram illustrating an example of a nurse call reception screen displayed on the mobile terminal device illustrated in Fig. 8. Fig. 18 is a diagram illustrating a near-death determination request screen displayed on the mobile terminal device illustrated in Fig. 8.

In the monitored person monitoring system MS having the above-described configuration, the devices SU, SV, SP, and TAA execute initialization of necessary parts and start operation when power is turned on. In the sensor device SU, the SU control unit 141, the action detection processing unit 142, the respiratory abnormality determination unit 143, the nurse call processing unit 144, and the SU streaming processing unit 145 are functionally configured in the SU control processing unit 14 by the execution of the control processing program. In the management server device SV, the SV control unit 211, the SV monitoring notification processing unit 222, and the clock unit 223 are functionally configured in the SV control processing unit 21, and the SV monitoring processing unit 2221 and the notification processing unit 2222 are functionally configured in the SV monitoring notification processing unit 222 by the execution of the control processing program. Then, in the mobile terminal device TAA, the TA control unit 321, the TA monitoring processing unit 322, the call processing unit 323, and the TA streaming processing unit 324 are functionally configured in the TA control processing unit 32 by the execution of the control processing program.

First, the operation of the sensor device SU will be described. The sensor device SU operates as follows at predetermined sampling intervals, for example, for each frame or every several frames to detect a predetermined motion in the monitored person Ob, detects the respiratory abnormality, and determines presence or absence of reception of the nurse call.

In Fig. 9, first, the sensor device SU acquires, by the SU control unit 141 of the SU control processing unit 14, one frame of image (image data) as the object image from the imaging unit 111 of the sensor unit 11 (S10).

Next, the sensor device SU executes, by the action detection processing unit 142 of the SU control processing unit 14, action detection processing of detecting the predetermined action in the monitored person Ob on the basis of the object image acquired in the processing S10 (S11). More specifically, the action detection processing unit 142 determines occurrence of the wake up, occurrence of the bed leaving, occurrence of the rolling off, and then occurrence of the fall.

Next, the sensor device SU determines, by the action detection processing unit 142, whether the predetermined action in the monitored person Ob has been detected in the action detection processing S11. As a result of the determination, in a case where the predetermined action has not been detected (No), the sensor device SU next executes processing S14, whereas in a case where the predetermined action has been detected (Yes), the sensor device SU executes the processing S14 after executing next processing S13.

In the processing S13, to notify the predetermined terminal device SP or TAA of the predetermined action detected in the processing S11 or S12 via the management server device SV, the sensor device SU transmits, by the action detection processing unit 142, the first event notification communication signal regarding detection of the predetermined action as the predetermined event to the management server device SV. More specifically, the action detection processing unit 142 transmits the first event notification communication signal that accommodates the sensor ID of its own device, the predetermined event (one or a plurality of the wake up, bed leaving, rolling off, and fall), the object images regarding the event to the management server device SV via the SU communication IF unit 15.

In the processing S14, the sensor device SU acquires, by the SU control unit 141 of the SU control processing unit 14, the measurement result from the respiration measurement unit 112 of the sensor unit 11 and stores the measurement result in the SU storage unit 16. The SU storage unit 16 stores at least the measurement result of the respiration measurement unit 112, which is necessary for detection of the respiratory abnormality of the monitored person Ob.

Next, the sensor device SU executes, by the respiratory abnormality determination unit 143 of the SU control processing unit 14, respiratory abnormality detection processing of detecting the respiratory abnormality of the monitored person Ob on the basis of the measurement result of the respiration measurement unit 112 acquired in the processing S14 (S15). More specifically, the respiratory abnormality determination unit 143 determines occurrence of the respiratory arrest, occurrence of the sleep apnea, occurrence of the slow respiration, occurrence of the tachypnea, occurrence of the hypopnea, occurrence of the hyperpnea, occurrence of the Kussmaul respiration, and occurrence of the Cheyne-Stokes respiration.

Next, the sensor device SU determines, by the respiratory abnormality determination unit 143, whether the respiratory abnormality of the monitored person Ob has been detected in the respiratory abnormality detection processing S15. As a result of the determination, in a case where the respiratory abnormality has not been detected (No), the sensor device SU next executes processing S18, whereas in a case where the respiratory abnormality has been detected (Yes), the sensor device SU executes the processing S18 after executing next processing S17.

In the processing S17, to notify the predetermined terminal device SP or TAA of the respiratory abnormality detected in the processing S14 or S15 via the management server device SV, the sensor device SU transmits, by the respiratory abnormality determination unit 143, the first event notification communication signal regarding detection of the respiratory abnormality as the predetermined event to the management server device SV. More specifically, the respiratory abnormality determination unit 143 transmits the first event notification communication signal that accommodates the sensor ID of its own device, the predetermined event (here the respiratory abnormality), and the object images regarding the event to the management server device SV via the SU communication IF unit 15.

In the processing S18, the sensor device SU determines, by the nurse call processing unit 144, whether the nurse call has been received. That is, while the processing S10 to S19 illustrated in Fig. 9 is repeatedly executed at the predetermined sampling intervals, whether the nurse call reception operation unit 13 has been operated is determined during a period from execution of the previous processing S18 to execution of the current processing S18. As a result of the determination, in a case where the nurse call reception operation unit 13 has not been operated and the nurse call has not been received (No), the sensor device SU terminates the present processing of this time, whereas in a case where the nurse call reception operation unit 13 has been operated and the nurse call has been received (Yes), the sensor device SU terminates the present processing after executing the next processing S19.

In the processing S19, to notify via the management server device SV the predetermined terminal device SP or TAA of the nurse call, reception of which has been determined in the processing S18, the sensor device SU transmits, by the nurse call processing unit 144, the first event notification communication signal regarding the nurse call to the management server device SV. More specifically, the nurse call processing unit 144 transmits the first event notification communication signal that accommodates the sensor ID of its own device and nurse call reception information to the management server device SV via the SU communication IF unit 15.

The sensor device SU operates as described above regarding each of detection of the predetermined action of the monitored person Ob, detection of the respiratory abnormality, and reception of the nurse call.

Next, the operation of the management server device SV will be described. Meanwhile, in Fig. 10, the management server device SV determines, by the SV control unit 221 of the SV control processing unit 22, whether the communication signal has been received in the SV communication IF unit 21 (S21). As a result of the determination, in a case where the communication signal has not been received (No), the management server SV returns the processing to S21. As a result of the determination, in a case where the communication signal has been received (Yes), the management server device SV executes the next processing S22. That is, the management server device SV waits for reception of the communication signal.

In the processing S22, the management server device SV determines, by the SV control unit 221, the type of the received communication signal. As a result of the determination, the management server device SV executes processing S27 after executing processing S23 in a case where the received communication signal is the first event notification communication signal (first event notification), executes the processing S27 after executing processing S24 in a case where the received communication signal is the near-death determination desire communication signal (desire near-death determination), executes the processing S27 after executing processing S25 in a case where the received communication signal is the desire for near-death notification communication signal (desire for near-death notification), and executes the processing S27 after executing processing S26 in a case where the received communication signal is none of the first event notification communication signal, the near-death determination desire communication signal, and the desire for near-death notification communication signal (others).

In the processing S23, the management server device SV processes, by the SV monitoring notification processing unit 222 of the SV control processing unit 22, the first event notification communication signal received from the sensor device SU in the processing S21.

More specifically, in Fig. 11, first, the SV monitoring notification processing unit 222 determines whether the event information accommodated in the first event notification communication signal received from the sensor device SU in the processing S21 is the respiratory abnormality (S231). As a result of the determination, in a case where the event information is not the respiratory abnormality (the event information is the type of the predetermined action or the nurse call, No), the SV monitoring notification processing unit 222 executes the processing S232 by the SV monitoring processing unit 2221. In a case where the event information is the respiratory abnormality (Yes), the SV monitoring notification processing unit 222 executes processing S233 by the SV monitoring processing unit 2221.

In this processing S232, the SV monitoring processing unit 2221 stores (records) the monitoring information regarding monitoring of the monitored person Ob accommodated in the first event notification communication signal received in the processing S21 to the SV monitoring information storage unit 231, and then executes processing S237.

Meanwhile, in the processing S233, the notification processing unit 2222 stores (records) the monitoring information regarding monitoring of the monitored person Ob accommodated in the first event notification communication signal received in the processing S21 to the SV monitoring information storage unit 231, similarly to the processing S232 of the SV monitoring processing unit 2221, and then executes processing S234.

In the processing S234, the notification processing unit 2222 obtains the notification condition on the basis of the respiratory abnormality accommodated in the first event notification communication signal received in the processing in S21. More specifically, in the present embodiment, the notification processing unit 2222 fetches, from the symptom history information storage unit 234, the symptom history information corresponding to the sensor ID (in other words, the monitored person Ob associated with the sensor device SU having the sensor ID) accommodated in the first event notification communication signal received in the processing S21. Next, the notification processing unit 2222 determines the notification condition on the basis of the respiratory abnormality and the fetched symptom history information. More specifically, the notification processing unit 2222 determines the condition B as the notification condition in a case of the respiratory abnormality and a case where the symptom history information of the monitored person Ob includes the past history of heart failure. The notification processing unit 2222 determines the condition A as the notification condition in a case of the respiratory abnormality and a case where the symptom history information of the monitored person Ob is another case excluding the case of the past history of heart failure.

Next, the notification processing unit 2222 determines the type of the notification condition obtained in the processing S234. As a result of the determination, in a case where the notification condition obtained in the processing S234 is the condition A(normal), the notification processing unit 2222 executes the processing S237, whereas in a case where the notification condition obtained in the processing S234 is the condition B (at emergency), the notification processing unit 2222 executes processing S236.

In the processing S236, the notification processing unit 2222 determines whether the preset predetermined time has passed from a transmission time of the previous near-death determination request communication signal stored in the SV storage unit 23 in processing S238 or S242 to be described below. As a result of the determination, in a case where the predetermined time has passed (Yes), the notification processing unit 2222 executes the processing S238. In a case where the predetermined time has not passed (No), the notification processing unit 2222 terminates the present processing S23 and executes the next processing S27. Note that, in this case, the notification processing unit 2222 may be configured to transmit the second event notification communication signal to the terminal device SP or TAA.

In the processing S237, the SV monitoring notification processing unit 222 selects (searches for), by the SV monitoring processing unit 2221 or the notification processing unit 2222, the notification destination corresponding to the sensor device SU that has transmitted the first event notification communication signal received in the processing S21 from the notification destination A field 2322 in the condition-based notification destination correspondence information table AT stored in the inter-device information storage unit 232, transmits the second event notification communication signal to the selected terminal device SP or TAA, terminates the present processing S23, and executes the next processing S27.

Then, in the processing S238, the notification processing unit 2222 selects (searches for) the first notification destination corresponding to the sensor device SU that has transmitted the first event notification communication signal received in the processing S21 from the notification destination B field 2323 of the condition-based notification destination correspondence information table AT stored in the inter-device information storage unit 232, transmits the near-death determination request communication signal to the selected external telephone ICT (mobile telephone TAB), acquires a current time from the clock unit 223, stores the acquired current time in association with the sensor ID (the monitored person Ob) accommodated in the first event notification communication signal received in the processing S21 as the transmission time of the near-death determination request communication signal in the SV storage unit 23, terminates the present processing S23, and executes the next processing S27.

Returning to Fig. 10, in the processing S24, the management server device SV processes, by the SV monitoring notification processing unit 222 of the SV control processing unit 22, the near-death determination desire communication signal received in the processing S21.

More specifically, referring to Fig. 12, first, the notification processing unit 2222 determines whether the preset predetermined time has passed from the transmission time of the previous near-death determination request communication signal stored in the SV storage unit 23 (S241). As a result of the determination, in a case where the predetermined time has passed (Yes), the notification processing unit 2222 executes the processing S242. In a case where the predetermined time has not passed (No), the notification processing unit 2222 executes processing S243.

Then, in the processing S242, the notification processing unit 2222 selects (searches for) the second notification destination corresponding to the sensor ID accommodated in the near-death determination desire communication signal received in the processing S21 from the notification destination B field 2323 of the condition-based notification destination correspondence information table AT stored in the inter-device information storage unit 232, transmits the near-death determination request communication signal to the selected external telephone ICT (mobile telephone TAB), acquires a current time from the clock unit 223, stores the acquired current time in association with the sensor ID (the monitored person Ob) accommodated in the near-death determination desire communication signal received in the processing S21 as the transmission time of the near-death determination request communication signal in the SV storage unit 23, terminates the present processing S24, and executes the next processing S27.

Meanwhile, in the processing S243, the notification processing unit 2222 transmits a communication signal (wait-to-see notification communication signal) for notifying that it is in a wait-to-see state to the transmission source of the near-death determination desire communication signal received in the processing S21, terminates the present processing S24, and executes the next processing S27. The wait-to-see notification communication signal accommodates the sensor ID accommodated in the near-death determination desire communication signal received in the processing S21 and a message notifying that it is in the wait-to-see state (for example, "in wait-to-see").

Returning to Fig. 10, in the processing S25, the management server device SV processes, by the SV monitoring notification processing unit 222 of the SV control processing unit 22, the desire for near-death notification communication signal received in the processing S21. More specifically, the notification processing unit 2222 selects (searches for) the notification destination C corresponding to the sensor ID accommodated in the desire for near-death notification communication signal received in the processing S21 from the notification destination C field 2324 of the condition-based notification destination correspondence information table AT stored in the inter-device information storage unit 232, transmits the near-death notification communication signal to the selected external telephone ICT (the fixed telephone TL in the present embodiment), and executes the next processing S27.

In the processing S26, the management server device SV executes, by the SV control processing unit 22, appropriate processing according to the communication signal received in the processing S21, and executes the next processing S27.

Then, in the processing S27 executed after the processing S23 and S26, the management server device SV determines, by the SV control processing unit 22, whether the operation is terminated (stopped). As a result of the determination, in a case where the operation is terminated (stopped) (Yes), the management server device SV terminates the present processing. On the other hand, in a case where the operation is not terminated (stopped) (No), the management server device SV returns the processing to the processing S21.

The management server device SV operates as described above regarding the first event notification communication signal, the near-death determination desire communication signal, and the desire for near-death notification communication signal.

Next, the operation of the terminal device SP or TAA will be described. Here, the operation of the mobile terminal device TAA will be representatively described. As described above, when the power is turned on and the operation is started, the mobile terminal device TAA accepts a log-in operation by the monitor (user) such as the nurse or the caretaker, and a standby screen for waiting for the communication signal of its own device is displayed on the TA display unit 36 by the TA monitoring processing unit 322. A standby screen 51 includes, for example, as illustrated in Fig. 14, a menu bar area 511 for displaying a menu bar, a standby main area 512 for displaying a message (for example, "no notification") notifying that it is on standby and an icon, a time area 513 for displaying the current time, a year/month/date/day area 514 for displaying today's year, month, date, and day, and a user name area 515 for displaying a user name who has currently logged-in to the mobile terminal device TA.

Then, in Fig. 13, the mobile terminal device TAA determines, by the TA control unit 321 of the TA control processing unit 32, whether the communication signal has been received in the TA communication IF unit 31 (S31). As a result of the determination, in a case where the communication signal has not been received (No), the mobile terminal device TAA returns the processing to S31. As a result of the determination, in a case where the communication signal has been received (Yes), the mobile terminal device TAA executes the next processing S32. That is, the mobile terminal device TAA waits for reception of the communication signal.

In the processing S32, the mobile terminal device TAA determines, by the TA control unit 321, the type of the received communication signal. As a result of the determination, the mobile terminal device TAA executes the next processing S33 in a case where the communication signal received in the processing S31 is the second event notification communication signal (second event notification), and executes processing S37 of performing appropriate processing according to the communication signal received in the processing S31 and then terminates the present processing in a case where the communication signal received in the processing S31 is not the second event notification communication signal (others).

In processing S33, the mobile terminal device TAA stores (records), by the TA monitoring processing unit 322 of the TA control processing unit 32, the monitoring information regarding monitoring of the monitored person Ob accommodated in the second event notification communication signal received from the management server device SV in the processing S31 to the TA monitoring information storage unit 331.

Next to the processing S33, the TA monitoring processing unit 322 displays, on the TA display unit 36, a screen according to each piece of the information accommodated in the second event notification communication signal received in the processing S31 (S34), and executes the next processing S35. More specifically, the TA monitoring processing unit 322 displays a first mode monitoring information screen 52a for displaying, on the TA display unit 36, pieces of information regarding the predetermined action illustrated in Fig. 15, for example, in a case where the event information accommodated in the second event notification communication signal received in the processing S31 is the predetermined action, displays a second mode monitoring information screen 52b for displaying, on the TA display unit 36, pieces of information regarding the respiratory abnormality illustrated in Fig. 16, for example, in a case where the event information accommodated in the second event notification communication signal received in the processing S31 is the respiratory abnormality, and displays, on the TA display unit 36, a nurse call reception screen 53 illustrated in Fig. 17, for example, in a case where the event information accommodated in the second event notification communication signal received in processing S31 is the nurse call.

The first mode monitoring information screen 52a is a screen for displaying the monitoring information of the monitored person Ob regarding the predetermined action. For example, as illustrated in Fig. 15, the first mode monitoring information screen 52a includes a menu bar area 511, a monitored person name area 521 for displaying the arrangement place of the sensor device SU having the sensor ID accommodated in the second event notification communication signal received in the processing S31 and the name of the monitored person Ob monitored by the sensor device SU having the sensor ID, a detection information display area 522 for displaying an elapsed time from the reception time of the second event notification communication signal received in the processing S31 (or a detection time of the predetermined action) and the event information (a detection result of the predetermined action) accommodated in the second event notification communication signal received in the processing S31, an image area 523 for displaying the images (that is, the object images imaged by the sensor device SU having the sensor ID) (here, the sill image) accommodated in the second event notification communication signal received in the processing S31, a "deal" button 524, a "talk" button 525, and a "watch LIVE" button 526.

To display the arrangement place of the sensor device SU and the name of the monitored person Ob in the monitored person name area 521, the TA sensor information storage unit 332 is searched for the arrangement place of the sensor device SU and the name of the monitored person Ob, using the sensor ID accommodated in the second event notification communication signal received in the processing S31 as a search key, and the arrangement place and the name of the monitored person Ob are displayed. In the detection information display area 522, the detection result (names of the wake up, bed leaving, rolling off, and fall in the present embodiment) accommodated in the second event notification communication signal received in the processing S31 may be displayed as it is. However, in the present embodiment, an icon symbolically representing the detection result is displayed. To display the icon, the TA storage unit 33 stores the actions and icons symbolically representing the actions in association with each other in advance. Note that the TA storage unit 33 stores the respiratory abnormality and an icon symbolically representing the respiratory abnormality in association with each other in advance. In the example illustrated in Fig. 15, a wake-up icon that symbolizes the wake up is displayed in the detection information display area 522. The "deal" button 524 is, on the first mode monitoring information screen 52a, a button for inputting, to the mobile terminal device TAA, implementation intention information indicating that the user of the mobile terminal device TAA has an intention to implement predetermined deal (handling) of lifesaving, nursing, care, or assistance with respect to the detection result displayed on the monitoring information screen 52a. The "talk" button 525 is a button for requesting a voice call and is a button for inputting an instruction to connect the sensor device SU of the sensor ID with the mobile terminal device TAA via the network NW in a callable manner. The "watch LIVE" button 526 is a button for requesting a live moving image and is a button for inputting an instruction to display a moving image imaged by the sensor device SU of the sensor ID.

The second mode monitoring information screen 52b is a screen for displaying the monitoring information of the monitored person Ob regarding the respiratory abnormality. The second mode monitoring information screen 52b includes, for example, as illustrated in Fig. 16, the menu bar area 511, the monitored person name area 521, the detection information display area 522, the image area 523, a "resume" button 527, a "desire near-death determination" button 528, and a "view respiration waveform" button 529. In the example illustrated in Fig. 16, a wake-up icon that symbolizes the respiratory abnormality is displayed in the detection information display area 522.

The "resume" button 527 is a button for inputting an instruction of continuation of monitoring the respiration of the monitored person Ob associated with the sensor device SU having the sensor ID accommodated in the second event notification communication signal received in the processing S31. The "desire near-death determination" button 528 is a button for instructing transfer of the respiratory abnormality accommodated in the second event notification communication signal received in the processing S31 and for inputting an instruction to obtain a request of determination as to whether the monitored person Ob associated with the sensor device SU having the sensor ID accommodated in the second event notification communication signal is at the last moment. The "view respiration waveform" button 529 is a button for inputting an instruction of display of the measurement result measured by the respiration measurement unit 112 of the sensor device SU having the sensor ID accommodated in the second event notification communication signal received in the processing S31.

The nurse call reception screen 53 is a screen for displaying reception of the nurse call. The nurse call reception screen 53 includes, for example, as illustrated in Fig. 17, the menu bar area 511, the monitored person name area 521, the detection information display area 522, a nurse call reception notification display area 531 for displaying a message notifying that the nurse call has been received (for example, "a nurse call"), the "deal" button 524, and the "talk" button 525. In the detection information display area 522 on the nurse call reception screen 53, only an elapsed time from the reception time of the second event notification communication signal received in the processing S31 (or a reception time when the nurse call has been received) is displayed.

Note that the nurse call reception screen 53 may further include the "watch LIVE" button 526.

Returning to Fig. 13, in the processing S35, the mobile terminal device TAA determines, by the TA control processing unit 32, whether an input operation has been received on a touch panel provided with the TA input unit 35 and the TA display unit 36 (S35). As a result of the determination, in a case where the input operation has not been received (No), the mobile terminal devices TAA returns the processing to S35. On the other hand, as a result of the determination, in a case where the input operation has been received (Yes), the mobile terminal device TAA executes the next processing S36.

In the processing S36, the mobile terminal device TAA executes, by the TA control processing unit 32, appropriate processing according to the content of the input operation and terminates the present processing.

For example, when the mobile terminal device TAA receives, by the TA control processing unit 32, the input operation of the "deal" button 524 from the TA input unit 35 constituting the touch panel (that is, when receiving the deal intention), the mobile terminal device TAA first adds the fact that the "deal" has been received to the monitoring information of the monitored person Ob currently displayed on the TA display unit 36 and stores the monitoring information to the TA monitoring information storage unit 331. More specifically, the TA control processing unit 32 registers the deal/resume flag "1" representing reception of deal to the deal/resume field 3316 of the record in which the monitoring information of the monitored person Ob currently displayed on the TA display unit 36 is registered (here, the record in which the monitoring information accommodated in the second event notification communication signal received in the processing S31 is registered) in the TA monitoring information table MT-TA stored in the TA monitoring information storage unit 331. Then, the TA control processing unit 32 transmits, to the management server device SV, the communication signal (deal reception notification communication signal) that accommodates the sensor ID corresponding to the monitoring information of the monitored person Ob displayed on the TA display unit 36 and information (deal reception information) indicating that the "deal" has been received. The management server device SV that has received the deal reception notification communication signal registers, by the SV control processing unit 22, the sensor ID accommodated in the received deal reception notification communication signal to the sensor ID field 2311, and searches for, by the SV control processing unit 22, the record to which the deal/resume flag "0" is to be registered in the deal/resume field 2316 and registers the deal/resume flag "1" to the deal/resume field 2316 of the searched record, and then transmits a communication signal (deal reception informing communication signal) that accommodates the sensor ID accommodated in the received deal reception notification communication signal and the deal reception information to the terminal devices SP and TAA by broadcast communication. With the transmission, the fact that the "deal" has been received with respect to the sensor ID corresponding to the monitoring information of the monitored person Ob displayed on the TA display unit 36 is synchronized by the terminal devices SP and TAA.

Further, for example, when the mobile terminal device TAA receives, by the TA control processing unit 32, the input operation of the "talk" button 525, the mobile terminal device TAA transmits, by the call processing unit 323, a communication signal (call request communication signal) that accommodates information indicating that a voice call is requested to the sensor device SU that monitors the monitored person Ob displayed on the TA display unit 36, and is connected with the sensor device SU that has responded to the request via the network NW in a voice callable manner. With the connection, the voice call becomes available between the mobile terminal device TAA and the sensor device SU. Note that, when the mobile terminal device TAA receives, by the TA control processing unit 32, an input operation of an "end" button (illustration is omitted) that is a button for inputting an instruction to terminate the voice call, the mobile terminal device TAA transmits, by the call processing unit 323, a communication signal (call termination communication signal) that accommodates information indicating that the termination of the voice call is requested to the sensor device SU that monitors the monitored person Ob displayed on the TA display unit 36. With the transmission, the voice call between the mobile terminal device TAA and the sensor device SU is terminated.

Further, for example, when the mobile terminal device TAA receives, by the TA control processing unit 32, the input operation of the "watch LIVE" button 526, the mobile terminal device TAA transmits, by the TA streaming processing unit 324, a communication signal (moving image distribution request communication signal) that accommodates information indicating that distribution of a live moving image is requested to the sensor device SU that monitors the monitored person Ob currently displayed on the TA display unit 36, is connected with the sensor device SU that has responded to the request via the network NW in a moving image downloadable manner, and receives distribution of the live moving image from the sensor device SU and displays the distributed moving image on the TA display unit 36 by streaming reproduction. On the monitoring information screen 52 that displays the live moving image, the moving image is displayed in the image area 523 and a "LIVE end" button (illustration is omitted) is displayed in place of the "watch LIVE" button 526. Thereby, the live moving image is displayed in the mobile terminal device TAA. The "LIVE end" button (not illustrated) is a button for requesting termination of the moving image, and is a button for inputting an instruction to terminate (stop) the distribution of the moving image imaged by the sensor device SU of the sensor ID to terminate (stop) the display. When the mobile terminal device TAA receives, by the TA control processing unit 32, the input operation of the "LIVE end" button, the mobile terminal device TAA transmits, by the TA streaming processing unit 324, a communication signal (moving image distribution termination communication signal) that accommodates information indicating that termination of the moving image distribution is requested to the sensor device SU that monitors the monitored person Ob currently displayed on the TA display unit 36, and displays a still image on the TA display unit 36. With the transmission, the mobile terminal device TAA terminates the display of the live moving image.

Further, for example, when the mobile terminal device TAA receives, by the TA control processing unit 32, the input operation of the "resume" button 524 from the TA input unit 35 constituting the touch panel, the mobile terminal device TAA adds the fact that the "resume" has been received to the monitoring information of the monitored person Ob currently displayed on the TA display unit 36 and stores the monitoring information to the TA monitoring information storage unit 331. More specifically, the TA control processing unit 32 registers the deal/resume flag "1 " representing reception of resume to the deal/resume field 3316 of the record that registers the monitoring information of the monitored person Ob currently displayed on the TA display unit 36 (here, the record in which the monitoring information accommodated in the second event notification communication signal received in the processing S31 is registered) in the TA monitoring information table MT-TA stored in the TA monitoring information storage unit 331. Then, the TA control processing unit 32 transmits, to the management server device SV, the communication signal (resume reception notification communication signal) that accommodates the sensor ID corresponding to the monitoring information of the monitored person Ob displayed on the TA display unit 36 and information (resume reception information) indicating that the "resume" has been received. The management server device SV that has received the resume reception notification communication signal registers, by the SV control processing unit 22, the sensor ID accommodated in the received resume reception notification communication signal to the sensor ID field 2311, and searches for, by the SV control processing unit 22, the record to which the deal/resume flag "0" is to be registered in the deal/resume field 2316 and registers the deal/resume flag "1" to the deal/resume field 2316 of the searched record, and then transmits a communication signal (resume reception informing communication signal) that accommodates the sensor ID accommodated in the received resume reception notification communication signal and the resume reception information to the terminal devices SP and TAA by broadcast communication. With the transmission, the fact that the "resume" has been received with respect to the sensor ID corresponding to the monitoring information of the monitored person Ob displayed on the TA display unit 36 is synchronized by the terminal devices SP and TAA.

Further, for example, when the mobile terminal device TAA receives, by the TA control processing unit 32, the input operation of the "view respiration waveform" button 529, the mobile terminal device TAA transmits a communication signal (respiration waveform request communication signal) that accommodates information indicating that the measurement result of the respiration measurement unit 112 in the sensor device SU that monitors the monitored person Ob displayed on the TA display unit 36 is requested, to the management server device SV. The management server device SV, which has responded to the request, sequentially acquires the measurement result of the respiration measurement unit 112 from the sensor device SU that monitors the monitored person Ob via the network NW, and responds to the mobile terminal device TAA while storing (recording) the sequentially acquired measurement result of the respiration measurement unit 112 in the SV storage unit 23. As a result, in the mobile terminal device TAA, the respiration waveform of the monitored person Ob according to the measurement result of the respiration measurement unit 112 relayed by the management server device SV is displayed in a newly opened window, overlapping with the image area 523, and a "respiration waveform end" button (illustration is omitted) is displayed in place of the "view respiration waveform" button 529. Thereby, the live respiration waveform is displayed in the mobile terminal device TAA. The "respiration waveform end" button (not illustrated) is a button for requesting termination of viewing of the respiration waveform, and is a button for inputting an instruction to cause the management server device SV to terminate (stop) the relay of the measurement result of the respiration measurement unit 112 measured by the sensor device SU of the sensor ID to terminate (stop) the display. When the mobile terminal device TAA receives, by the TA control processing unit 32, the input operation of the "respiration waveform end" button, the mobile terminal device TAA transmits a communication signal (respiration waveform termination communication signal) that accommodates information indicating that termination of the relay of the respiration waveform of the monitored person Ob currently displayed on the TA display unit 36 is requested, to the management server device SV, and closes the window. With the transmission, the mobile terminal device TAA terminates the display of the live respiration waveform.

Further, for example, when the mobile terminal device TAA receives, by the TA control processing unit 32, the input operation of the "desire near-death determination" button 528, the mobile terminal device TAA transmits, by the TA monitoring processing unit 322, the near-death determination desire communication signal for the monitored person Ob displayed on the TA display unit 36 to the management server device SV. The near-death determination desire communication signal accommodates the sensor ID accommodated in the second event notification communication signal received in the processing S31, the event information (respiratory abnormality) and the object images regarding the event information, and an instruction to transfer the respiratory abnormality as a desire for the near-death determination. When receiving the near-death determination desire communication signal, the management server device SV selects (searches for), by the notification processing unit 2222 of the SV monitoring notification processing unit 222, the second notification destination corresponding to the sensor ID accommodated in the received near-death determination desire communication signal from the notification destination B field 2323 of the condition-based notification destination correspondence information table AT stored in the inter-device information storage unit 232, and transmits the near-death determination request communication signal to the selected external telephone ICT (the mobile telephone TAB handled by the medical personnel in the present embodiment). The near-death determination request communication signal accommodates the name of the monitored person Ob corresponding to the sensor ID accommodated in the near-death determination desire communication signal, the sensor ID accommodated in the near-death determination desire communication signal, the event information (respiratory abnormality) and the object images regarding the event information, and a predetermined message (for example, a "near-death determination request") requesting the determination as to whether the monitored person Ob is at the last moment, as described above.

When receiving the near-death determination request communication signal, the mobile telephone TAB of the external telephone ICT handled by the medical personnel such as a doctor displays a near-death determination request screen for displaying the monitoring information of the monitored person Ob regarding the respiratory abnormality and the request of determination as to whether the monitored person Ob is at the last moment.

A near-death determination request screen 54 includes, for example, as illustrated in Fig. 18, the monitored person name area 521, the detection information display area 522, the image area 523, the "resume" button 527, a "desire near-death notification" button 541, and the "view respiration waveform" button 529. On the near-death determination request screen 54, only the monitored person name is displayed in the monitored person name area 521, and only an icon of the respiratory abnormality is displayed in the detection information display area 522. In the present embodiment, such a near-death determination request screen 54 is displayed in the mobile telephone TAB, and thus the near-death determination request communication signal accommodates data of files described in a markup language such as hyper text markup language (HTML) and the sensor ID to configure the near-death determination request screen 54 illustrated in Fig. 18, using the name of the monitored person Ob, the icon of the respiratory abnormality, and the object images and the messages (for example, the "near-death determination request").

When the medical personnel handling the mobile telephone TAB determines that the monitored person Ob is the last moment on the near-death determination request screen 54, the medical personnel operates the "desire near-death notification" 541 on the near-death determination request screen 54. When receiving the input operation of the "desire near-death notification" button 541, the mobile telephone TAB returns the desire for near-death notification communication signal for obtaining notification of the message indicating that the monitored person Ob is at the last moment to the management server device SV. The desire for near-death notification communication signal accommodates the sensor ID accommodated in the received near-death determination request communication signal and the second instruction notifying the message indicating that the monitored person Ob is at the last moment. When receiving the desire for near-death notification communication signal in the processing S21, the management server device SV sequentially executes the above processing S22 and S25, selects (searches for) the notification destination C corresponding to the sensor ID accommodated in the desire for received desire for near-death notification communication signal by the notification processing unit 2222 in the processing S25 from the notification destination C field 2324 of the condition-based notification destination correspondence information table AT stored in the inter-device information storage unit 232, and transmits the near-death notification communication signal to the selected external telephone ICT (the fixed telephone TL in the present embodiment). With the transmission, the near-death notification communication signal is transmitted to the notification destination C associated with the monitored person Ob, and the fact that the monitored person Ob is at the last moment is notified to the person who handles the external telephone ICT (the fixed telephone TL in the present embodiment) that has received the near-death notification communication signal.

The mobile terminal device TA operates as described above with respect to the second event notification communication signal and the like.

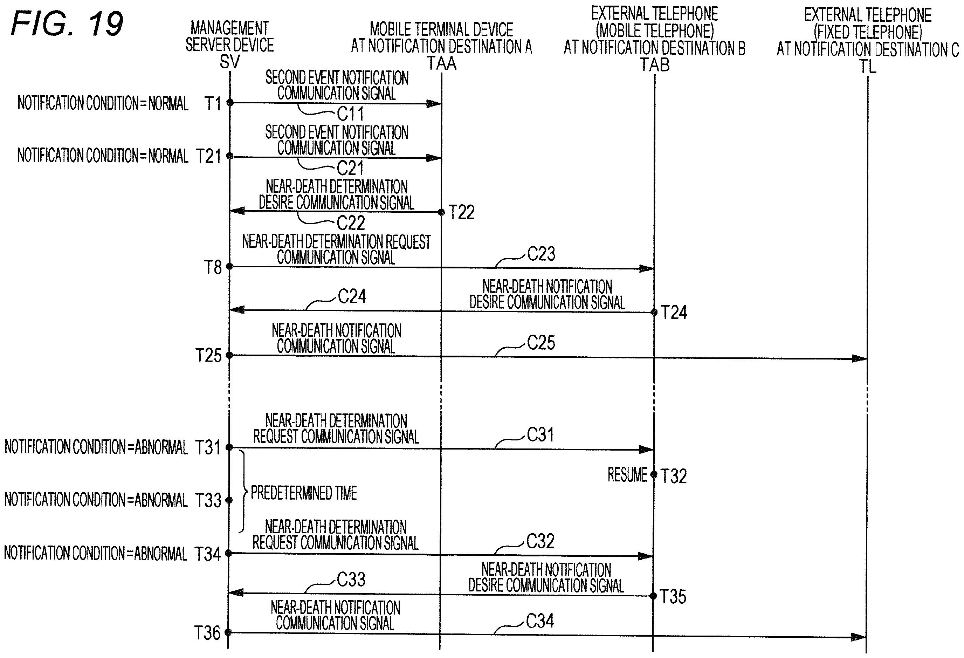

Here, the operation of the entire monitored person monitoring system MS provided with the sensor device SU, the management server device SV, and the terminal devices SP and TAA respectively operating as described above will be described by way of an example. Fig. 19 is a sequence diagram illustrating an operation example of the monitored person monitoring system illustrated in Fig. 1.

In Fig. 19, the sensor device SU repeatedly executes the processing S10 to S19 described above with reference to Fig. 9 at predetermined time intervals. In the repetitive execution, when detecting the respiratory abnormality, the sensor device SU transmits a first event communication signal regarding the respiratory abnormality to the management server device SV. When receiving the first event communication signal regarding the respiratory abnormality from the sensor device SU in the above processing S21, the management server device SV sequentially executes the above processing S22 and S23. In this processing S23, the above processing S231, S233, S234, and S235 are sequentially executed. When the normal condition is determined as the condition A in the processing S235, the processing S237 is executed, and the second event notification communication signal is transmitted to the mobile terminal device TAA at the notification destination A at time T11 (C11).

Further, when detecting the respiratory abnormality, the sensor device SU transmits the first event communication signal regarding the respiratory abnormality to the management server device SV. The management server device SV operates similarly to the above time T11, and the second event notification communication signal is transmitted to the mobile terminal device TAA at the notification destination A at time T21 (C21). The mobile terminal device TAA at the notification destination A, which has received the second event notification communication signal in the processing S31, sequentially executes the processing S32 to S34 and displays the second mode monitoring information screen 52b regarding the second event notification communication signal received in the processing S31 on the TA display unit 36. The monitor, who handles the mobile terminal device TAA at the notification destination A, views the image of the monitored person Ob displayed in the image area 523 on the second mode monitoring information screen 52b regarding the second event notification communication signal and wants to seek determination as to whether the monitored person Ob is at the last moment from the medical personnel, and operates the "desire near-death determination" button 528. When receiving the input operation of the "desire near-death determination" button 528 in the processing S35 at time T22, the mobile terminal device TAA at the notification destination A transmits the near-death determination desire communication signal to the management server device SV in the processing S36 (C22). When receiving the near-death determination desire communication signal from the mobile terminal device TAA at the notification destination A in the processing S21, the management server device SV sequentially executes the processing S22 to S24. In the processing S24, the processing S241 to S242 are sequentially executed, and the near-death determination request communication signal is transmitted to the mobile telephone TAB at the notification destination B at time T23 (C23). The mobile telephone TAB at the notification destination B, which has received the near-death determination request communication signal, displays the near-death determination request screen 54 regarding the received near-death determination request communication signal. When the medical personnel who handles the mobile telephone TAB at the notification destination B views the image of the monitored person Ob displayed in the image area 523 on the near-death determination request screen 54 regarding the near-death determination request communication signal, the respiration waveform displayed according to the input operation of the "view respiration waveform" button 529, and the like, and determines that the monitored person Ob is at the last moment, the medical personnel operates the "desire near-death notification" button 541. The mobile telephone TAB that has received the input operation of the "desire near-death notification" button 541 at time T24 transmits the desire for near-death notification communication signal to the management server device SV (C24). When receiving the desire for near-death notification communication signal from the mobile telephone TAB at the notification destination B in the above processing S21, the management server device SV sequentially executes the above processing S22 and S25. At time T24 in the processing S25, the management server device SV selects (searches for) the notification destination C corresponding to the sensor ID accommodated in the desire for near-death notification communication signal received in the processing S21 from the notification destination C field 2324 of the condition-based notification destination correspondence information table AT stored in the inter-device information storage unit 232, and transmits the near-death notification communication signal to the selected external telephone ICT (the fixed telephone TL in the present embodiment) (C25).

Meanwhile, when detecting the respiratory abnormality, the sensor device SU transmits the first event communication signal regarding the respiratory abnormality to the management server device SV. When receiving the first event communication signal regarding the respiratory abnormality from the sensor device SU in the above processing S21, the management server device SV sequentially executes the above processing S22 and S23. In this processing S23, the above processing S231, S233, S234, and S235 are sequentially executed. When the normal condition is determined as the condition B in the processing S235, the processing S236 and S238 are sequentially executed, and the near-death determination request communication signal is transmitted to the mobile telephone TAB at the notification destination B at time T31 (C31). The mobile telephone TAB at the notification destination B, which has received the near-death determination request communication signal, displays the near-death determination request screen 54 regarding the received near-death determination request communication signal. When the medical personnel who handles the mobile telephone TAB at the notification destination B views the image of the monitored person Ob displayed on the image area 523 on the near-death determination request screen 54 regarding the near-death determination request communication signal, the respiration waveform displayed according to an input operation of the "view respiration waveform" button 529, and the like, and determines that the monitored person Ob is not at the last moment, the medical personnel operates the "resume" button 527 at time T32. Therefore, monitoring of the respiration is continued.

Further, when detecting the respiratory abnormality, the sensor device SU transmits the first event communication signal regarding the respiratory abnormality to the management server device SV. When receiving the first event communication signal regarding the respiratory abnormality from the sensor device SU in the above processing S21, the management server device SV sequentially executes the above processing S22 and S23. In this processing S23, the above processing S231, S233, S234, and S235 are sequentially executed, and when the normal condition is determined as the condition B in the processing S235, the management server device SV executes the processing S236. When it is determined that a predetermined time has not passed from the previous transmission in the near-death determination request communication signal in the processing S236, transmission of the near-death determination request communication signal is suspended (that is, not transmitted) at time T33.

Further, when detecting the respiratory abnormality, the sensor device SU transmits the first event communication signal regarding the respiratory abnormality to the management server device SV. When receiving the first event communication signal regarding the respiratory abnormality from the sensor device SU in the above processing S21, the management server device SV sequentially executes the above processing S22 and S23. In this processing S23, the above processing S231, S233, S234, and S235 are sequentially executed, and when the normal condition is determined as the condition B in the processing S235, the management server device SV executes the processing S236. When it is determined that a predetermined time has passed from the previous transmission in the near-death determination request communication signal in the processing S236, the near-death determination request communication signal is transmitted to the mobile telephone TAB at the notification destination B at time T34 (C32). The mobile telephone TAB at the notification destination B, which has received the near-death determination request communication signal, displays the near-death determination request screen 54 regarding the received near-death determination request communication signal. When the medical personnel who handles the mobile telephone TAB at the notification destination B views the image of the monitored person Ob displayed in the image area 523 on the near-death determination request screen 54 regarding the near-death determination request communication signal, the respiration waveform displayed according to the input operation of the "view respiration waveform" button 529, and the like, and determines that the monitored person Ob is at the last moment, the medical personnel operates the "desire near-death notification" button 541. At time T35, the mobile telephone TAB, which has received the input operation of the "desire near-death notification" button 541, transmits the desire for near-death notification communication signal to the management server device SV (C33). When receiving the desire for near-death notification communication signal from the mobile telephone TAB at the notification destination B in the above processing S21, the management server device SV sequentially executes the above processing S22 and S25. At time T36 in the processing S25, the management server device SV selects (searches for) the notification destination C corresponding to the sensor ID accommodated in the desire for near-death notification communication signal received in the processing S21 from the notification destination C field 2324 of the condition-based notification destination correspondence information table AT stored in the inter-device information storage unit 232, and transmits the near-death notification communication signal to the selected external telephone ICT (the fixed telephone TL in the present embodiment) (C34).

As described above, in the monitored person monitoring system MS, the sensor device SU and the management server device SV as an example of the monitored person monitoring device, and the monitored person monitoring method incorporated in the aforementioned system and devices in the present embodiment, in a case where the instruction to forward the determination result is received from the first notification destination to which the determination result of the respiratory abnormality determination unit 143 (that is, the respiratory abnormality in the present embodiment) has been notified, the determination result of the respiratory abnormality determination unit 143 is re-notified to the notification destination different from the first notification destination. Therefore, the determination result of the respiratory abnormality determination unit 143 is considered by a plurality of notification destinations, and the last moment of the monitored person Ob can be more appropriately determined, and thus the contact can be more appropriately conducted. Therefore, the monitored person monitoring system MS, the monitored person monitoring device (the sensor device SU and the management server device SV), and the monitored person monitoring method can support the contact at the last moment of the monitored person Ob.

The monitored person monitoring system MS, the monitored person monitoring device (the sensor device SU and the management server device SV), and the monitored person monitoring method notify a third notification destination (the notification destination C in the present embodiment) associated with the monitored person Ob of the message indicating that the monitored person Ob is at the last moment, thereby to support the contact at the last moment of the monitored person Ob.

The monitored person monitoring system MS, the monitored person monitoring device (the sensor device SU and the management server device SV), and the monitored person monitoring method obtain the notification condition further on the basis of the symptom history information associated with the monitored person Ob, thereby to more appropriately obtain the notification condition.

When respiratory abnormality is further measured in a case where the monitored person has a past history of heart failure, a possibility that the monitored person is at the last moment is high. The monitored person monitoring system MS, the monitored person monitoring device (the sensor device SU and the management server device SV), and the monitored person monitoring method can more appropriately obtain the notification condition because the symptom history information is the past history of heart failure.

The monitored person monitoring system MS, the monitored person monitoring device (the sensor device SU and the management server device SV), and the monitored person monitoring method include the "view respiration waveform" button 529, thereby to more appropriately determine whether requesting the determination as to whether the monitored person Ob is at the last moment or whether the monitored person Ob is at the last moment by reference to the measurement result of the respiration measurement unit.

In the monitored person monitoring system MS, the monitored person monitoring device (the sensor device SU and the management server device SV), and the monitored person monitoring method, the first notification destination is the caretaker or the medical personnel, and the second notification destination is the medical personnel. Therefore, whether the monitored person Ob is at the last moment can be primarily determined by the caretaker, and when the determination is difficult for the caretaker, whether the monitored person Ob is at the last moment can be further secondarily determined by the medical personnel. Therefore, the monitored person monitoring system MS, the monitored person monitoring device (the sensor device SU and the management server device SV), and the monitored person monitoring method can more appropriately determine whether the monitored person Ob is at the last moment, and the burden on the medical personnel can be reduced as compared with a case where the determination result of the respiratory abnormality determination unit 143 is simultaneously notified to the caretaker and the medical personnel.

Note that, in the above embodiment, in the case where the notification condition is the condition B, the monitored person Ob has the respiratory abnormality and the symptom history information of the monitored person Ob includes the past history of heart failure. However, the notification condition may be determined as the condition B in the following cases in place of or in addition to the above case.

First, in a case where there is second respiratory abnormality within a predetermined period, the notification condition is determined as the condition B. This is because the possibility of the last moment is high if respiratory abnormality happens twice within the predetermined period. In such a case, the symptom history information is the respiratory abnormality of the monitored person determined by the respiratory abnormality determination unit within the predetermined period, and the symptom history information storage unit 234 stores the respiratory abnormality and the reception time of the first event notification communication signal regarding the respiratory abnormality in association with the sensor ID (monitored person Ob). The predetermined period is appropriately set to, for example, 30 minutes, 1 hour, 3 hours, or 6 hours.

Secondly, in a case where the respiratory abnormality is a so-called low-ventilatory system (for example, slow respiration or hypopnea) and the vital is abnormal, the notification condition is determined as the condition B. The vital is, for example, the blood oxygen level (SpO₂), and the decrease of the blood oxygen level is determined as abnormal (the blood oxygen level equal to or lower than a preset threshold value). In such a case, the sensor device SU further includes a vital measurement unit 113 that measures the vital in the sensor unit 11, as illustrated by the broke line in Fig. 2, and further transmits a measurement result of the vital measurement unit 113 to the management server device SV. The vital measurement unit 113 includes, for example, a pulse oximeter or the like for measuring the blood oxygen level.

Further, in the above embodiment, the notification processing unit 2222 may be configured to perform the re-notification processing with the second notification destination as the first notification destination. For example, the notification destination A includes a plurality of notification destinations and the notification destination A field 2322 includes a plurality of subfields so as to register the plurality of notification destinations. Then, the notification destination is sequentially selected in every re-notification processing from among the plurality of notification destinations at the notification destination A. According to this configuration, in a case where a new caretaker is unable to determine whether requesting determination as to whether the monitored person Ob is at the last moment, the caretaker can seek determination from a more experienced caretaker such as a trainer caretaker or a chief caretaker. Further, for example, the notification destination B includes a plurality of notification destinations and the notification destination B field 2323 includes a plurality of subfields so as to register the plurality of notification destinations. Then, the notification destination is sequentially selected in every re-notification processing from among the plurality of notification destinations at the notification destination B. According to this configuration, in a case where a new doctor is unable to determine whether the monitored person Ob is at the last moment, the doctor can seek determination from a more experienced doctor such as a trainer doctor or a chief doctor. Further, for example, according to this configuration, in a case where a doctor who specializes in the field A is unable to determine whether the monitored person Ob is at the last moment, the doctor can seek determination from a doctor who specializes in another field B, which is different from the field A.

Although the present specification discloses the techniques of various aspects as described above, the main techniques among them are summarized below.

A monitored person monitoring device according to one aspect includes a respiration measurement unit provided in association with a monitored person that is an object to be monitored and configured to measure respiration of the monitored person, a respiratory abnormality determination unit configured to determine whether the respiration of the monitored person is abnormal on the basis of a measurement result of the respiration measurement unit, a condition-based notification destination information storage unit configured to store a plurality of notification destinations classified according to predetermined criteria as condition-based notification destination information in association with a predetermined notification condition, and a notification processing unit configured to obtain a notification condition on the basis of a determination result of the respiratory abnormality determination unit, select a notification destination corresponding to the obtained notification condition as a first notification destination from the condition-based notification destination information stored in the condition-based notification destination information storage unit, and notify the selected first notification destination of the determination result of the respiratory abnormality determination unit. Then, in a case where the notification processing unit receives an instruction to forward the determination result of the respiratory abnormality determination unit from the first notification destination to which the determination result of the respiratory abnormality determination unit has been notified, the notification processing unit selects another notification destination excluding the notification destination to which the determination result of the respiratory abnormality determination unit has already been notified as a second notification destination from among the plurality of notification destinations, and performs re-notification processing of notifying the selected second notification destination of the determination result of the respiratory abnormality determination unit. Favorably, in the above-described monitored person monitoring device, the notification processing unit performs the re-notification processing to the second notification destination as the first notification destination. Favorably, the above-described monitored person monitoring device includes a sensor device and a management server device communicatively connected with the sensor device, and the sensor device includes the respiration measurement unit, the respiratory abnormality determination unit, and a respiratory abnormality notification processing unit that notifies the management server device of the determination result of the respiratory abnormality determination unit, and the management server device includes the condition-based notification destination information storage unit and the notification processing unit.

In a case where such a monitored person monitoring device receives an instruction to forward the determination result from the first notification destination to which the determination result of the respiratory abnormality determination unit has been notified, the monitored person monitoring device re-notifies a notification destination different from the first notification destination of the determination result of the respiratory abnormality determination unit. Therefore, the determination result of the respiratory abnormality determination unit is considered by a plurality of notification destinations, and the last moment of the monitored person can be more appropriately determined, and thus the contact can be more appropriately conducted. Therefore, the monitored person monitoring device can support the contact at the last moment of the monitored person.

In another aspect, in the above-described monitored person monitoring device, the plurality of notification destinations includes a third notification destination associated with the monitored person and to which a message indicating a last moment is notified, and in a case where the notification processing unit receives a second instruction to notify the message indicating that the monitored person is at the last moment from the first or second notification destination to which the determination result of the respiratory abnormality determination unit has been notified, the notification processing unit notifies the third notification destination associated with the monitored person of the message.

Such a monitored person monitoring device notifies the third notification destination associated with the monitored person of the message indicating that the monitored person is at the last moment, thereby to support the contact at the last moment of the monitored person.

In another aspect, in the above-described monitored person monitoring device, a symptom history information storage unit configured to store a history of symptoms as symptom history information in association with the monitored person is further included, and the notification processing unit obtains the notification condition further on the basis of the symptom history information associated with the monitored person.

Such a monitored person monitoring device obtains the notification condition further on the basis of the symptom history information associated with the monitored person, thereby to more appropriately obtain the notification condition.

In another aspect, in the above-described monitored person monitoring device, the symptom history information is a past history of heart failure.

When respiratory abnormality is further measured in a case where the monitored person has a past history of heart failure, a possibility that the monitored person is at the last moment is high. The monitored person monitoring device can more appropriately obtain the notification condition because the symptom history information is the past history of heart failure.

In another aspect, in the above-described monitored person monitoring device, the symptom history information is respiratory abnormality of the monitored person determined by the respiratory abnormality determination unit within a predetermined period.

When the respiratory abnormality is measured again within the predetermined period, the possibility that the monitored person is at the last moment is high. The monitored person monitoring device can more appropriately obtain the notification condition because the symptom history information is the respiratory abnormality of the monitored person determined by the respiratory abnormality determination unit within a predetermined period.

In another aspect, in the above-described monitored person monitoring device, a respiration measurement result notification processing unit configured to notify the second notification destination of the measurement result of the respiration measurement unit in a case where the respiration measurement result notification processing unit receives a third instruction to notify the measurement result of the respiration measurement unit from the second notification destination is further included. Favorably, in the above-described monitored person monitoring device, the respiration measurement result notification processing unit notifies the first notification destination of the measurement result of the respiration measurement unit in a case where the respiration measurement result notification processing unit further receives a fourth instruction to notify the measurement result of the respiration measurement unit from the first notification processing unit.

Such a monitored person monitoring device includes the respiration measurement result notification processing unit, whereby the second notification destination can more appropriately determine whether the monitored person is at the last moment by reference to the measurement result of the respiration measurement unit.

In another aspect, in the above-described monitored person monitoring device, the plurality of notification destinations is composed of three notification destinations including a third notification destination associated with a caretaker, a medical personnel, and the monitored person, and which notifies a message indicating a last moment, the first notification destination is the caretaker or the medical personnel, and the second notification destination is the medical personnel.

Such a monitored person monitored person monitoring device can primarily determine whether the monitored person is at the last moment by the caretaker, and can further secondarily determine whether the monitored person is at the last moment by the medical personnel in a case where the caretaker has a difficulty in making a determination. Therefore, the monitored person monitoring device can more appropriately determine whether the monitored person is at the last moment, and the burden on the medical personnel can be reduced as compared with a case where the determination result of the respiratory abnormality determination unit is simultaneously notified to the caretaker and the medical personnel.

A monitored person monitoring method according to another aspect includes a respiration measurement step of measuring respiration of a monitored person that is an object to be measured, the step being executed in association with the monitored person, a respiratory abnormality determination step of determining whether the respiration of the monitored person is abnormal on the basis of a measurement result of the respiration measurement step, and a notification processing step of selecting a notification destination corresponding to a notification condition obtained on the basis of a determination result of the respiratory abnormality determination step as a first notification destination from condition-based notification destination information in which a plurality of notification destinations classified according to predetermined criteria is associated with a predetermined notification condition, and notifying the selected first notification destination of the determination result of the respiratory abnormality determination step. Then, in the notification processing step, in a case where an instruction to forward the determination result of the respiratory abnormality determination step is received from the first notification destination to which the determination result of the respiratory abnormality determination step has been notified, another notification destination excluding the notification destination to which the determination result of the respiratory abnormality determination step has already been notified is selected as a second notification destination from among the plurality of notification destinations, and re-notification processing of notifying the selected second notification destination of the determination result of the respiratory abnormality determination step is performed.

In such a monitored person monitoring method, in a case where an instruction to forward the determination result is received from the first notification destination to which the determination result of the respiratory abnormality determination step has been notified, the determination result of the respiratory abnormality determination step is re-notified to a notification destination different from the first notification destination. Therefore, the determination result of the respiratory abnormality determination step is considered by a plurality of notification destinations, and the last moment of the monitored person can be more appropriately determined, and thus the contact can be more appropriately conducted. Therefore, the monitored person monitoring method can support the contact at the last moment of the monitored person.

A monitored person monitoring system according to another aspect is a monitored person monitoring system including a terminal device, and a monitored person monitoring device communicatively connected to the terminal device, wherein the monitored person monitoring device is any one of the above-described monitored person monitoring devices.

Since such a monitored person monitoring system includes any of the above-described monitored person monitoring devices, thereby to support the contact at the last moment of the monitored person.

This application is based on Japanese Patent Application No. 2016-115042 filed on June 9, 2016, the contents of which are hereby incorporated herein by reference.

To express the present invention, while the present invention has been appropriately and fully described through the embodiments with reference to the drawings in the foregoing, it should be recognized that those skilled in the art can easily modify and/or improve the above-described embodiments. Therefore, it is construed that modified forms and improved forms are included in the scope of claims as long as the modified forms and improved forms implemented by those skilled in the art do not depart from the scope of claims described in the claims.

### Industrial Applicability

According to the present invention, a monitored person monitoring device, a monitored person monitoring method, and a monitored person monitoring system for monitoring a monitored person who is an object to be monitored can be provided.

## Claims

1. A monitored person monitoring device comprising:
a respiration measurement unit provided in association with a monitored person that is an object to be monitored and configured to measure respiration of the monitored person;
a respiratory abnormality determination unit configured to determine whether the respiration of the monitored person is abnormal on the basis of a measurement result of the respiration measurement unit;
a condition-based notification destination information storage unit configured to store a plurality of notification destinations classified according to predetermined criteria as condition-based notification destination information in association with a predetermined notification condition; and
a notification processing unit configured to obtain a notification condition on the basis of a determination result of the respiratory abnormality determination unit, select a notification destination corresponding to the obtained notification condition as a first notification destination from the condition-based notification destination information stored in the condition-based notification destination information storage unit, and notify the selected first notification destination of the determination result of the respiratory abnormality determination unit, wherein
in a case where the notification processing unit receives an instruction to forward the determination result of the respiratory abnormality determination unit from the first notification destination to which the determination result of the respiratory abnormality determination unit has been notified, the notification processing unit selects another notification destination excluding the notification destination to which the determination result of the respiratory abnormality determination unit has already been notified as a second notification destination from among the plurality of notification destinations, and performs re-notification processing of notifying the selected second notification destination of the determination result of the respiratory abnormality determination unit.

2. The monitored person monitoring device according to claim 1, wherein
the plurality of notification destinations includes a third notification destination associated with the monitored person and to which a message indicating a last moment is notified, and
in a case where the notification processing unit receives a second instruction to notify the message indicating that the monitored person is at the last moment from the first or second notification destination to which the determination result of the respiratory abnormality determination unit has been notified, the notification processing unit notifies the third notification destination associated with the monitored person of the message.

3. The monitored person monitoring device according to claim 1 or 2, further comprising
a symptom history information storage unit configured to store a history of symptoms as symptom history information in association with the monitored person, wherein
the notification processing unit obtains the notification condition further on the basis of the symptom history information associated with the monitored person.

4. The monitored person monitoring device according to claim 3, wherein
the symptom history information is a past history of heart failure.

5. The monitored person monitoring device according to claim 3, wherein
the symptom history information is respiratory abnormality of the monitored person determined by the respiratory abnormality determination unit within a predetermined period.

6. The monitored person monitoring device according to any one of claims 1 to 5, further comprising
a respiration measurement result notification processing unit configured to notify the second notification destination of the measurement result of the respiration measurement unit in a case where the respiration measurement result notification processing unit receives a third instruction to notify the measurement result of the respiration measurement unit from the second notification destination.

7. The monitored person monitoring device according to any one of claims 1 to 6, wherein
the plurality of notification destinations is composed of three notification destinations including a third notification destination associated with a caretaker, a medical personnel, and the monitored person, and which notifies a message indicating a last moment,
the first notification destination is the caretaker or the medical personnel, and
the second notification destination is the medical personnel.

8. A monitored person monitoring method comprising:
a respiration measurement step of measuring respiration of a monitored person that is an object to be measured, the step being executed in association with the monitored person;
a respiratory abnormality determination step of determining whether the respiration of the monitored person is abnormal on the basis of a measurement result of the respiration measurement step; and
a notification processing step of selecting a notification destination corresponding to a notification condition obtained on the basis of a determination result of the respiratory abnormality determination step as a first notification destination from condition-based notification destination information in which a plurality of notification destinations classified according to predetermined criteria is associated with a predetermined notification condition, and notifying the selected first notification destination of the determination result of the respiratory abnormality determination step, wherein
in the notification processing step, in a case where an instruction to forward the determination result of the respiratory abnormality determination step is received from the first notification destination to which the determination result of the respiratory abnormality determination step has been notified, another notification destination excluding the notification destination to which the determination result of the respiratory abnormality determination step has already been notified is selected as a second notification destination from among the plurality of notification destinations, and re-notification processing of notifying the selected second notification destination of the determination result of the respiratory abnormality determination step is performed.

9. A monitored person monitoring system comprising:
a terminal device; and
a monitored person monitoring device communicatively connected to the terminal device, wherein
the monitored person monitoring device is the monitored person monitoring device according to any one of claims 1 to 7.
